# EUROPEAN PATENT SPECIFICATION

(11) **EP 3 436 078 B1**
(45) Date of publication and mention of the grant of the patent: **27.10.2021**
(21) Application number: 17776753.0
(22) Date of filing: 31.03.2017
(51) Int. Cl.: A61K 48/00, C07K 14/71, C07K 14/715, A61P 21/00, C12N 15/63, C12N 15/861, C12N 15/85, C12N 15/86, A61P 25/28

(54) **METHODS AND COMPOSITIONS FOR THE TREATMENT OF ALS**
VERFAHREN UND ZUSAMMENSETZUNGEN ZUR BEHANDLUNG VON ALS
MÉTHODES ET COMPOSITIONS POUR LE TRAITEMENT DE LA SLA

(30) Priority: 31.03.2016 US 201662315988 P; 14.12.2016 US 201662433985 P; 14.12.2016 US 201662433987 P
(43) Date of publication of application: 06.02.2019
(73) Proprietor: University of Cincinnati, Cincinnati, OH 45206 (US)
(72) Inventor: MACLENNAN, Alexander John, Cincinnati 45206 Ohio (US)
(74) Representative: Moore, Michael Richard
(86) International application number: PCT/US2017/025315
(87) International publication number: WO 2017/173234

(56) References cited:
- WO-A1-98/11213
- WO-A1-2015/143078
- WO-A2-01/55172
- WO-A2-01/94605
- WO-A2-97/33912
- US-A1- 2014 335 054
- THOMAS W GOULD ET AL: "Motor neuron trophic factors: Therapeutic use in ALS?", BRAIN RESEARCH REVIEWS, ELSEVIER, NL, vol. 67, no. 1, 18 October 2010 (2010-10-18), pages 1-39, XP028374655, ISSN: 0165-0173, DOI: 10.1016/J.BRAINRESREV.2010.10.003 [retrieved on 2010-10-21]
- LEE et al.: "Muscle Ciliary Neurotrophic Factor Receptor a Promotes Axonal Regeneration And Functional Recovery Following Peripheral Nerve Lesion", J Comp Neurol. Author Manuscript, vol. 521, no. 13, 1 September 2014 (2014-09-01), pages 1-33, XP055579177,
- FORGER et al.: "Cardiotrophin-Like Cytokine/Cytokine-Like Factor 1 Is An Essential Trophic Factor For Lumbar And Facial Motoneurons In Vivo", The Journal of Neuroscience, vol. 23, no. 26, 1 October 2003 (2003-10-01), pages 8854-8858, XP055579183,

### SEQUENCE LISTING

<110> University of Cincinnati
<120> METHODS AND COMPOSITIONS FOR THE TREATMENT OF ALS
<130> 10738-535
<150> 62/433985
   <151> 2016-03-31
<150> 62433985
   <151> 2016-12-14
<150> 62433987
   <151> 2016-12-14
<160> 8
<170> PatentIn version 3.5
<210> 1
   <211> 6525
   <212> DNA
   <213> Rattus norvegicus
<220>
   <221> misc_feature
   <223> Vector comprising rat CNTFR alpha DNA sequence
<400> 1
   gggggggggg ggggggggtt ggccactccc tctctgcgcg ctcgctcgct cactgaggcc      60
   gggcgaccaa aggtcgcccg acgcccgggc tttgcccggg cggcctcagt gagcgagcga     120
   gcgcgcagag agggagtggc caactccatc actaggggtt cctagatctg aattcggtac     180
   cctagttatt aatagtaatc aattacgggg tcattagttc atagcccata tatggagttc     240
   cgcgttacat aacttacggt aaatggcccg cctggctgac cgcccaacga cccccgccca     300
   ttgacgtcaa taatgacgta tgttcccata gtaacgccaa tagggacttt ccattgacgt     360
   caatgggtgg actatttacg gtaaactgcc cacttggcag tacatcaagt gtatcatatg     420
   ccaagtacgc cccctattga cgtcaatgac ggtaaatggc ccgcctggca ttatgcccag     480
   tacatgacct tatgggactt tcctacttgg cagtacatct acgtattagt catcgctatt     540
   accatggtcg aggtgagccc cacgttctgc ttcactctcc ccatctcccc cccctcccca     600
   cccccaattt tgtatttatt tattttttaa ttattttgtg cagcgatggg ggcggggggg     660
   gggggggggc gcgcgccagg cggggcgggg cggggcgagg ggcggggcgg ggcgaggcgg     720
   agaggtgcgg cggcagccaa tcagagcggc gcgctccgaa agtttccttt tatggcgagg     780
   cggcggcggc ggcggcccta taaaaagcga agcgcgcggc gggcgggagt cgctgcgacg     840
   ctgccttcgc cccgtgcccc gctccgccgc cgcctcgcgc cgcccgcccc ggctctgact     900
   gaccgcgtta ctcccacagg tgagcgggcg ggacggccct tctcctccgg gctgtaatta     960
   gcgcttggtt taatgacggc ttgtttcttt tctgtggctg cgtgaaagcc ttgaggggct 1020
   ccgggagggc cctttgtgcg ggggggagcg gctcgggggg tgcgtgcgtg tgtgtgtgcg 1080
   tggggagcgc cgcgtgcggc ccgcgctgcc cggcggctgt gagcgctgcg ggcgcggcgc 1140
   ggggctttgt gcgctccgca gtgtgcgcga ggggagcgcg gccgggggcg gtgccccgcg 1200
   gtgcgggggg ggctgcgagg ggaacaaagg ctgcgtgcgg ggtgtgtgcg tgggggggtg 1260
   agcagggggt gtgggcgcgg cggtcgggct gtaacccccc cctgcacccc cctccccgag 1320
   ttgctgagca cggcccggct tcgggtgcgg ggctccgtac ggggcgtggc gcggggctcg 1380
   ccgtgccggg cggggggtgg cggcaggtgg gggtgccggg cggggcgggg ccgcctcggg 1440
   ccggggaggg ctcgggggag gggcgcggcg gcccccggag cgccggcggc tgtcgaggcg 1500
   cggcgagccg cagccattgc cttttatggt aatcgtgcga gagggcgcag ggacttcctt 1560
   tgtcccaaat ctgtgcggag ccgaaatctg ggaggcgccg ccgcaccccc tctagcgggc 1620
   gcggggcgaa gcggtgcggc gccggcagga aggaaatggg cggggagggc cttcgtgcgt 1680
   cgccgcgccg ccgtcccctt ctccctctcc agcctcgggg ctgtccgcgg ggggacggct 1740
   gccttcgggg gggacggggc agggcggggt tcggcttctg gcgtgtgacc ggcggctcta 1800
   gagcctctgc taaccatgtt catgccttct tctttttcct acagctcctg ggcaacgtgc 1860
   tggttattgt gctgtctcat cattttggca aagaattcct cgaagatcta ggcctgcagg 1920
   cggccgctct agaactagtg gatcccccca acacatcatc ccaggaagac tttggtctgc 1980
   agaggaggat aatattgatg tgcttggaga gcatctggtg gtaacgagat ggctgcttct 2040
   gtcccttggg cctgctgtgc tgtgcttgcc gctgccgctg ccgctgtcta cacccagaaa 2100
   cacagtccac aggaggcacc ccatgttcag tatgagcgtc tgggcacaga tgtgacgctg 2160
   ccatgtggga cagcaagttg ggacgcagct gtgacctgga gggtaaatgg aacagatctg 2220
   gcccctgacc tgctcaatgg ctctcagctg atactacgaa gcttagaact gggccacagt 2280
   ggcctgtatg cctgtttcca ccgtgactcc tggcacctgc gccaccaagt ccttctgcat 2340
   gtgggtttgc cgcctcggga acccgtgctc agctgccgtt ccaacactta ccccaagggc 2400
   ttctactgca gctggcacct gtccgccccc acctacatcc ccaatacctt caatgtgact 2460
   gtactgcatg gctccaaaat gatggtctgt gagaaggacc cagccctcaa gaaccgctgt 2520
   cacattcggt acatgcacct gttctcaacc atcaagtaca aggtctccat aagtgtcagc 2580
   aacgccttgg gtcacaacac cacggctatc accttcgacg aattcaccat tgtgaagccc 2640
   gatcctccag aaaatgtggt ggcccggcca gtgcccagca acccccgtcg actggaggtg 2700
   acatggcaga ccccctcaac ttggcctgat cccgaatcct ttccactcaa gttttttctg 2760
   cgctaccggc ctctcatcct ggatcaatgg cagcatgtgg agctctcgaa tggcacagcc 2820
   cacaccatca cggatgccta tgctgggaag gagtacatca tccaggtggc cgccaaggac 2880
   aatgagattg ggacatggag tgactggagt gtggctgctc acgccacacc ctggactgag 2940
   gaaccacggc atctcaccac tgaagcccag gcccccgaga ccacgaccag caccaccagc 3000
   tccttggcac ccccacccac cacgaagatc tgtgaccccg gggagctcag cagcggcgga 3060
   ggaccctcca tacccttctt gaccagtgtc cctgtcactc tggtcctggc tgccgctgct 3120
   gccacagcca acaatctcct gatctgagcc ctgcatccca tgaggacacg ccagacgcct 3180
   acagaggagc aggaggccgg agctgagcct gcagaccccg gtttctattt tgcacacggg 3240
   caggaggggg ctgcaggaat tcgatatcaa gcttatcgat accgtcgact agagctcgct 3300
   gatcagcctc gactgtgcct tctagttgcc agccatctgt tgtttgcccc tcccccgtgc 3360
   cttccttgac cctggaaggt gccactccca ctgtcctttc ctaataaaat gaggaaattg 3420
   catcgcattg tctgagtagg tgtcattcta ttctgggggg tggggtgggg caggacagca 3480
   agggggagga ttgggaagac aatagcaggc atgctgggga gagatctagg aacccctagt 3540
   gatggagttg gccactccct ctctgcgcgc tcgctcgctc actgaggccg cccgggcaaa 3600
   gcccgggcgt cgggcgacct ttggtcgccc ggcctcagtg agcgagcgag cgcgcagaga 3660
   gggagtggcc aacccccccc cccccccccc tgcagccctg cattaatgaa tcggccaacg 3720
   cgcggggaga ggcggtttgc gtattgggcg ctcttccgct tcctcgctca ctgactcgct 3780
   gcgctcggtc gttcggctgc ggcgagcggt atcagctcac tcaaaggcgg taatacggtt 3840
   atccacagaa tcaggggata acgcaggaaa gaacatgtga gcaaaaggcc agcaaaaggc 3900
   caggaaccgt aaaaaggccg cgttgctggc gtttttccat aggctccgcc cccctgacga 3960
   gcatcacaaa aatcgacgct caagtcagag gtggcgaaac ccgacaggac tataaagata 4020
   ccaggcgttt ccccctggaa gctccctcgt gcgctctcct gttccgaccc tgccgcttac 4080
   cggatacctg tccgcctttc tcccttcggg aagcgtggcg ctttctcaat gctcacgctg 4140
   taggtatctc agttcggtgt aggtcgttcg ctccaagctg ggctgtgtgc acgaaccccc 4200
   cgttcagccc gaccgctgcg ccttatccgg taactatcgt cttgagtcca acccggtaag 4260
   acacgactta tcgccactgg cagcagccac tggtaacagg attagcagag cgaggtatgt 4320
   aggcggtgct acagagttct tgaagtggtg gcctaactac ggctacacta gaaggacagt 4380
   atttggtatc tgcgctctgc tgaagccagt taccttcgga aaaagagttg gtagctcttg 4440
   atccggcaaa caaaccaccg ctggtagcgg tggttttttt gtttgcaagc agcagattac 4500
   gcgcagaaaa aaaggatctc aagaagatcc tttgatcttt tctacggggt ctgacgctca 4560
   gtggaacgaa aactcacgtt aagggatttt ggtcatgaga ttatcaaaaa ggatcttcac 4620
   ctagatcctt ttaaattaaa aatgaagttt taaatcaatc taaagtatat atgagtaaac 4680
   ttggtctgac agttaccaat gcttaatcag tgaggcacct atctcagcga tctgtctatt 4740
   tcgttcatcc atagttgcct gactccccgt cgtgtagata actacgatac gggagggctt 4800
   accatctggc cccagtgctg caatgatacc gcgagaccca cgctcaccgg ctccagattt 4860
   atcagcaata aaccagccag ccggaagggc cgagcgcaga agtggtcctg caactttatc 4920
   cgcctccatc cagtctatta attgttgccg ggaagctaga gtaagtagtt cgccagttaa 4980
   tagtttgcgc aacgttgttg ccattgctac aggcatcgtg gtgtcacgct cgtcgtttgg 5040
   tatggcttca ttcagctccg gttcccaacg atcaaggcga gttacatgat cccccatgtt 5100
   gtgcaaaaaa gcggttagct ccttcggtcc tccgatcgtt gtcagaagta agttggccgc 5160
   agtgttatca ctcatggtta tggcagcact gcataattct cttactgtca tgccatccgt 5220
   aagatgcttt tctgtgactg gtgagtactc aaccaagtca ttctgagaat agtgtatgcg 5280
   gcgaccgagt tgctcttgcc cggcgtcaat acgggataat accgcgccac atagcagaac 5340
   tttaaaagtg ctcatcattg gaaaacgttc ttcggggcga aaactctcaa ggatcttacc 5400
   gctgttgaga tccagttcga tgtaacccac tcgtgcaccc aactgatctt cagcatcttt 5460
   tactttcacc agcgtttctg ggtgagcaaa aacaggaagg caaaatgccg caaaaaaggg 5520
   aataagggcg acacggaaat gttgaatact catactcttc ctttttcaat attattgaag 5580
   catttatcag ggttattgtc tcatgagcgg atacatattt gaatgtattt agaaaaataa 5640
   acaaataggg gttccgcgca catttccccg aaaagtgcca cctgacgtct aagaaaccat 5700
   tattatcatg acattaacct ataaaaatag gcgtatcacg aggccctttc gtctcgcgcg 5760
   tttcggtgat gacggtgaaa acctctgaca catgcagctc ccggagacgg tcacagcttg 5820
   tctgtaagcg gatgccggga gcagacaagc ccgtcagggc gcgtcagcgg gtgttggcgg 5880
   gtgtcggggc tggcttaact atgcggcatc agagcagatt gtactgagag tgcaccatat 5940
   gcggtgtgaa ataccgcaca gatgcgtaag gagaaaatac cgcatcagga aattgtaaac 6000
   gttaatattt tgttaaaatt cgcgttaaat ttttgttaaa tcagctcatt ttttaaccaa 6060
   taggccgaaa tcggcaaaat cccttataaa tcaaaagaat agaccgagat agggttgagt 6120
   gttgttccag tttggaacaa gagtccacta ttaaagaacg tggactccaa cgtcaaaggg 6180
   cgaaaaaccg tctatcaggg cgatggccca ctacgtgaac catcacccta atcaagtttt 6240
   ttggggtcga ggtgccgtaa agcactaaat cggaacccta aagggagccc ccgatttaga 6300
   gcttgacggg gaaagccggc gaacgtggcg agaaaggaag ggaagaaagc gaaaggagcg 6360
   ggcgctaggg cgctggcaag tgtagcggtc acgctgcgcg taaccaccac acccgccgcg 6420
   cttaatgcgc cgctacaggg cgcgtcgcgc cattcgccat tcaggctacg caactgttgg 6480
   gaagggcgat cggtgcgggc ctcttcgcta ttacgccagg ctgca 6525
<210> 2
   <211> 372
   <212> PRT
   <213> Rattus norvegicus
<220>
   <221> misc**_**feature
   <223> Rat CNTFR alpha amino acid sequence
<400> 2
   Met Ala Ala Ser Val Pro Trp Ala Cys Cys Ala Val Leu Ala Ala Ala
   1               5                   10                  15
   Ala Ala Ala Val Tyr Thr Gln Lys His Ser Pro Gln Glu Ala Pro His
               20                  25                  30
   Val Gln Tyr Glu Arg Leu Gly Thr Asp Val Thr Leu Pro Cys Gly Thr
           35                  40                  45
   Ala Ser Trp Asp Ala Ala Val Thr Trp Arg Val Asn Gly Thr Asp Leu
       50                  55                  60
   Ala Pro Asp Leu Leu Asn Gly Ser Gln Leu Ile Leu Arg Ser Leu Glu
   65                  70                  75                  80
   Leu Gly His Ser Gly Leu Tyr Ala Cys Phe His Arg Asp Ser Trp His
                   85                  90                  95
   Leu Arg His Gln Val Leu Leu His Val Gly Leu Pro Pro Arg Glu Pro
               100                 105                 110
   Val Leu Ser Cys Arg Ser Asn Thr Tyr Pro Lys Gly Phe Tyr Cys Ser
           115                 120                 125
   Trp His Leu Ser Ala Pro Thr Tyr Ile Pro Asn Thr Phe Asn Val Thr
       130                 135                 140
   Val Leu His Gly Ser Lys Met Met Val Cys Glu Lys Asp Pro Ala Leu
   145                 150                 155                 160
   Lys Asn Arg Cys His Ile Arg Tyr Met His Leu Phe Ser Thr Ile Lys
                   165                 170                 175
   Tyr Lys Val Ser Ile Ser Val Ser Asn Ala Leu Gly His Asn Thr Thr
               180                 185                 190
   Ala Ile Thr Phe Asp Glu Phe Thr Ile Val Lys Pro Asp Pro Pro Glu
           195                 200                 205
   Asn Val Val Ala Arg Pro Val Pro Ser Asn Pro Arg Arg Leu Glu Val
   210 215 220
   Thr Trp Gln Thr Pro Ser Thr Trp Pro Asp Pro Glu Ser Phe Pro Leu
   225 230 235 240
   Lys Phe Phe Leu Arg Tyr Arg Pro Leu Ile Leu Asp Gln Trp Gln His
   245 250 255
   Val Glu Leu Ser Asp Gly Thr Ala His Thr Ile Thr Asp Ala Tyr Ala
   260 265 270
   Gly Lys Glu Tyr Ile Ile Gln Val Ala Ala Lys Asp Asn Glu Ile Gly
   275 280 285
   Thr Trp Ser Asp Trp Ser Val Ala Ala His Ala Thr Pro Trp Thr Glu
   290 295 300
   Glu Pro Arg His Leu Thr Thr Glu Ala Gln Ala Pro Glu Thr Thr Thr
   305 310 315 320
   Ser Thr Thr Ser Ser Leu Ala Pro Pro Pro Thr Thr Lys Ile Cys Asp
   325 330 335
   Pro Gly Glu Leu Ser Ser Gly Gly Gly Pro Ser Ile Pro Phe Leu Thr
   340 345 350
   Ser Val Pro Val Thr Leu Val Leu Ala Ala Ala Ala Ala Thr Ala Asn
   355 360 365
   Asn Leu Leu Ile
   370
<210> 3
   <211> 372
   <212> PRT
   <213> Homo sapiens
<220>
   <221> misc**_**feature
   <223> Human CNTFR alpha amino acid sequence
<400> 3
   Met Ala Ala Pro Val Pro Trp Ala Cys Cys Ala Val Leu Ala Ala Ala
   1 5 10 15
   Ala Ala Val Val Tyr Ala Gln Arg His Ser Pro Gln Glu Ala Pro His
   20 25 30
   Val Gln Tyr Glu Arg Leu Gly Ser Asp Val Thr Leu Pro Cys Gly Thr
           35                  40                  45
   Ala Asn Trp Asp Ala Ala Val Thr Trp Arg Val Asn Gly Thr Asp Leu
       50                  55                  60
   Ala Pro Asp Leu Leu Asn Gly Ser Gln Leu Val Leu His Gly Leu Glu
   65                  70                  75                  80
   Leu Gly His Ser Gly Leu Tyr Ala Cys Phe His Arg Asp Ser Trp His
                   85                  90                  95
   Leu Arg His Gln Val Leu Leu His Val Gly Leu Pro Pro Arg Glu Pro
               100                 105                 110
   Val Leu Ser Cys Arg Ser Asn Thr Tyr Pro Lys Gly Phe Tyr Cys Ser
           115                 120                 125
   Trp His Leu Pro Thr Pro Thr Tyr Ile Pro Asn Thr Phe Asn Val Thr
       130                 135                 140
   Val Leu His Gly Ser Lys Ile Met Val Cys Glu Lys Asp Pro Ala Leu
   145                 150                 155                 160
   Lys Asn Arg Cys His Ile Arg Tyr Met His Leu Phe Ser Thr Ile Lys
                   165                 170                 175
   Tyr Lys Val Ser Ile Ser Val Ser Asn Ala Leu Gly His Asn Ala Thr
               180                 185                 190
   Ala Ile Thr Phe Asp Glu Phe Thr Ile Val Lys Pro Asp Pro Pro Glu
           195                 200                 205
   Asn Val Val Ala Arg Pro Val Pro Ser Asn Pro Arg Arg Leu Glu Val
       210                 215                 220
   Thr Trp Gln Thr Pro Ser Thr Trp Pro Asp Pro Glu Ser Phe Pro Leu
   225                 230                 235                 240
   Lys Phe Phe Leu Arg Tyr Arg Pro Leu Ile Leu Asp Gln Trp Gln His
                   245                 250                 255
   Val Glu Leu Ser Asp Gly Thr Ala His Thr Ile Thr Asp Ala Tyr Ala
               260                 265                 270
   Gly Lys Glu Tyr Ile Ile Gln Val Ala Ala Lys Asp Asn Glu Ile Gly
           275                 280                 285
   Thr Trp Ser Asp Trp Ser Val Ala Ala His Ala Thr Pro Trp Thr Glu
       290                 295                 300
   Glu Pro Arg His Leu Thr Thr Glu Ala Gln Ala Ala Glu Thr Thr Thr
   305                 310                 315                 320
   Ser Thr Thr Ser Ser Leu Ala Pro Pro Pro Thr Thr Lys Ile Cys Asp
                   325                 330                 335
   Pro Gly Glu Leu Gly Ser Gly Gly Gly Pro Ser Ala Pro Phe Leu Val
               340                 345                 350
   Ser Val Pro Ile Thr Leu Ala Leu Ala Ala Ala Ala Ala Thr Ala Ser
           355                 360                 365
   Ser Leu Leu Ile
       370
<210> 4
   <211> 372
   <212> PRT
   <213> Mus musculus
<220>
   <221> misc**_**feature
   <223> Mouse CNTFR alpha amino acid sequence
<400> 4
   Met Ala Ala Ser Val Pro Trp Ala Cys Cys Ala Val Leu Ala Ala Ala
   1               5                   10                  15
   Ala Ala Ala Val Tyr Thr Gln Lys His Ser Pro Gln Glu Ala Pro His
               20                  25                  30
   Val Gln Tyr Glu Arg Leu Gly Ala Asp Val Thr Leu Pro Cys Gly Thr
           35                  40                  45
   Ala Ser Trp Asp Ala Ala Val Thr Trp Arg Val Asn Gly Thr Asp Leu
       50                  55                  60
   Ala Pro Asp Leu Leu Asn Gly Ser Gln Leu Ile Leu Arg Ser Leu Glu
   65                  70                  75                  80
   Leu Gly His Ser Gly Leu Tyr Ala Cys Phe His Arg Asp Ser Trp His
                   85                  90                  95
   Leu Arg His Gln Val Leu Leu His Val Gly Leu Pro Pro Arg Glu Pro
               100                 105                 110
   Val Leu Ser Cys Arg Ser Asn Thr Tyr Pro Lys Gly Phe Tyr Cys Ser
           115                 120                 125
   Trp His Leu Pro Thr Pro Thr Tyr Ile Pro Asn Thr Phe Asn Val Thr
       130                 135                 140
   Val Leu His Gly Ser Lys Ile Met Val Cys Glu Lys Asp Pro Ala Leu
   145                 150                 155                 160
   Lys Asn Arg Cys His Ile Arg Tyr Met His Leu Phe Ser Thr Ile Lys
                   165                 170                 175
   Tyr Lys Val Ser Ile Ser Val Ser Asn Ala Leu Gly His Asn Thr Thr
               180                 185                 190
   Ala Ile Thr Phe Asp Glu Phe Thr Ile Val Lys Pro Asp Pro Pro Glu
           195                 200                 205
   Asn Val Val Ala Arg Pro Val Pro Ser Asn Pro Arg Arg Leu Glu Val
       210                 215                 220
   Thr Trp Gln Thr Pro Ser Thr Trp Pro Asp Pro Glu Ser Phe Pro Leu
   225                 230                 235                 240
   Lys Phe Phe Leu Arg Tyr Arg Pro Leu Ile Leu Asp Gln Trp Gln His
                   245                 250                 255
   Val Glu Leu Ser Asp Gly Thr Ala His Thr Ile Thr Asp Ala Tyr Ala
               260                 265                 270
   Gly Lys Glu Tyr Ile Ile Gln Val Ala Ala Lys Asp Asn Glu Ile Gly
           275                 280                 285
   Thr Trp Ser Asp Trp Ser Val Ala Ala His Ala Thr Pro Trp Thr Glu
       290                 295                 300
   Glu Pro Arg His Leu Thr Thr Glu Ala Gln Ala Pro Glu Thr Thr Thr
   305                 310                 315                 320
   Ser Thr Thr Ser Ser Leu Ala Pro Pro Pro Thr Thr Lys Ile Cys Asp
                   325                 330                 335
   Pro Gly Glu Leu Gly Ser Gly Gly Gly Pro Ser Ile Leu Phe Leu Thr
               340                 345                 350
   Ser Val Pro Val Thr Leu Val Leu Ala Ala Ala Ala Ala Thr Ala Asn
   355 360 365
   Asn Leu Leu Ile
   370
<210> 5
   <211> 225
   <212> PRT
   <213> Homo sapiens
<220>
   <221> misc**_**feature
   <223> Human CLC amino acid sequence
<400> 5
   Met Asp Leu Arg Ala Gly Asp Ser Trp Gly Met Leu Ala Cys Leu Cys
   1 5 10 15
   Thr Val Leu Trp His Leu Pro Ala Val Pro Ala Leu Asn Arg Thr Gly
   20 25 30
   Asp Pro Gly Pro Gly Pro Ser Ile Gln Lys Thr Tyr Asp Leu Thr Arg
   35 40 45
   Tyr Leu Glu His Gln Leu Arg Ser Leu Ala Gly Thr Tyr Leu Asn Tyr
   50 55 60
   Leu Gly Pro Pro Phe Asn Glu Pro Asp Phe Asn Pro Pro Arg Leu Gly
   65 70 75 80
   Ala Glu Thr Leu Pro Arg Ala Thr Val Asp Leu Glu Val Trp Arg Ser
   85 90 95
   Leu Asn Asp Lys Leu Arg Leu Thr Gln Asn Tyr Glu Ala Tyr Ser His
   100 105 110
   Leu Leu Cys Tyr Leu Arg Gly Leu Asn Arg Gln Ala Ala Thr Ala Glu
   115 120 125
   Leu Arg Arg Ser Leu Ala His Phe Cys Thr Ser Leu Gln Gly Leu Leu
   130 135 140
   Gly Ser Ile Ala Gly Val Met Ala Ala Leu Gly Tyr Pro Leu Pro Gln
   145 150 155 160
   Pro Leu Pro Gly Thr Glu Pro Thr Trp Thr Pro Gly Pro Ala His Ser
   165 170 175
   Asp Phe Leu Gln Lys Met Asp Asp Phe Trp Leu Leu Lys Glu Leu Gln
   180 185 190
   Thr Trp Leu Trp Arg Ser Ala Lys Asp Phe Asn Arg Leu Lys Lys Lys
   195 200 205
   Met Gln Pro Pro Ala Ala Ala Val Thr Leu His Leu Gly Ala His Gly
   210 215 220
   Phe
   225
<210> 6
   <211> 225
   <212> PRT
   <213> Mus musculus
<220>
   <221> misc**_**feature
   <223> Mouse CLC amino acid sequence
<400> 6
   Met Asp Leu Arg Ala Gly Asp Ser Trp Gly Met Leu Ala Cys Leu Cys
   1 5 10 15
   Thr Val Leu Trp His Leu Pro Ala Val Pro Ala Leu Asn Arg Thr Gly
   20 25 30
   Asp Pro Gly Pro Gly Pro Ser Ile Gln Lys Thr Tyr Asp Leu Thr Arg
   35 40 45
   Tyr Leu Glu His Gln Leu Arg Ser Leu Ala Gly Thr Tyr Leu Asn Tyr
   50 55 60
   Leu Gly Pro Pro Phe Asn Glu Pro Asp Phe Asn Pro Pro Arg Leu Gly
   65 70 75 80
   Ala Glu Thr Leu Pro Arg Ala Thr Val Asn Leu Glu Val Trp Arg Ser
   85 90 95
   Leu Asn Asp Arg Leu Arg Leu Thr Gln Asn Tyr Glu Ala Tyr Ser His
   100 105 110
   Leu Leu Cys Tyr Leu Arg Gly Leu Asn Arg Gln Ala Ala Thr Ala Glu
   115 120 125
   Leu Arg Arg Ser Leu Ala His Phe Cys Thr Ser Leu Gln Gly Leu Leu
   130 135 140
   Gly Ser Ile Ala Gly Val Met Ala Thr Leu Gly Tyr Pro Leu Pro Gln
   145                 150                 155                 160
   Pro Leu Pro Gly Thr Glu Pro Ala Trp Ala Pro Gly Pro Ala His Ser
                   165                 170                 175
   Asp Phe Leu Gln Lys Met Asp Asp Phe Trp Leu Leu Lys Glu Leu Gln
               180                 185                 190
   Thr Trp Leu Trp Arg Ser Ala Lys Asp Phe Asn Arg Leu Lys Lys Lys
           195                 200                 205
   Met Gln Pro Pro Ala Ala Ser Val Thr Leu His Leu Glu Ala His Gly
       210                 215                 220
   Phe
   225
<210> 7
   <211> 422
   <212> PRT
   <213> Homo sapiens
<220>
   <221> misc**_**feature
   <223> Human CLF amino acid sequence
<400> 7
<210> 8
   <211> 425
   <212> PRT
   <213> Mus musculus
<220>
   <221> misc**_**feature
   <223> Mouse CLF amino acid sequence
<400> 8
   Met Pro Ala Gly Arg Pro Gly Pro Val Ala Gln Ser Ala Arg Arg Pro
   1               5                   10                  15
   Pro Arg Pro Leu Ser Ser Leu Trp Ser Pro Leu Leu Leu Cys Val Leu
               20                  25                  30
   Gly Val Pro Arg Gly Gly Ser Gly Ala His Thr Ala Val Ile Ser Pro
           35                  40                  45
   Gln Asp Pro Thr Leu Leu Ile Gly Ser Ser Leu Gln Ala Thr Cys Ser
       50                  55                  60
   Ile His Gly Asp Thr Pro Gly Ala Thr Ala Glu Gly Leu Tyr Trp Thr
   65                  70                  75                  80
   Leu Asn Gly Arg Arg Leu Pro Ser Glu Leu Ser Arg Leu Leu Asn Thr
                   85                  90                  95
   Ser Thr Leu Ala Leu Ala Leu Ala Asn Leu Asn Gly Ser Arg Gln Gln
               100                 105                 110
   Ser Gly Asp Asn Leu Val Cys His Ala Arg Asp Gly Ser Ile Leu Ala
           115                 120                 125
   Gly Ser Cys Leu Tyr Val Gly Leu Pro Pro Glu Lys Pro Phe Asn Ile
       130                 135                 140
   Ser Cys Trp Ser Arg Asn Met Lys Asp Leu Thr Cys Arg Trp Thr Pro
   145                 150                 155                 160
   Gly Ala His Gly Glu Thr Phe Leu His Thr Asn Tyr Ser Leu Lys Tyr
                   165                 170                 175
   Lys Leu Arg Trp Tyr Gly Gln Asp Asn Thr Cys Glu Glu Tyr His Thr
               180                 185                 190
   Val Gly Pro His Ser Cys His Ile Pro Lys Asp Leu Ala Leu Phe Thr
           195                 200                 205
   Pro Tyr Glu Ile Trp Val Glu Ala Thr Asn Arg Leu Gly Ser Ala Arg
       210                 215                 220
   Ser Asp Val Leu Thr Leu Asp Val Leu Asp Val Val Thr Thr Asp Pro
   225                 230                 235                 240
   Pro Pro Asp Val His Val Ser Arg Val Gly Gly Leu Glu Asp Gln Leu
                   245                 250                 255
   Ser Val Arg Trp Val Ser Pro Pro Ala Leu Lys Asp Phe Leu Phe Gln
               260                 265                 270
   Ala Lys Tyr Gln Ile Arg Tyr Arg Val Glu Asp Ser Val Asp Trp Lys
           275                 280                 285
   Val Val Asp Asp Val Ser Asn Gln Thr Ser Cys Arg Leu Ala Gly Leu
       290                 295                 300
   Lys Pro Gly Thr Val Tyr Phe Val Gln Val Arg Cys Asn Pro Phe Gly
   305                 310                 315                 320
   Ile Tyr Gly Ser Lys Lys Ala Gly Ile Trp Ser Glu Trp Ser His Pro
                   325                 330                 335
   Thr Ala Ala Ser Thr Pro Arg Ser Glu Arg Pro Gly Pro Gly Gly Gly
               340                 345                 350
   Val Cys Glu Pro Arg Gly Gly Glu Pro Ser Ser Gly Pro Val Arg Arg
           355                 360                 365
   Glu Leu Lys Gln Phe Leu Gly Trp Leu Lys Lys His Ala Tyr Cys Ser
       370                 375                 380
   Asn Leu Ser Phe Arg Leu Tyr Asp Gln Trp Arg Ala Trp Met Gln Lys
   385                 390                 395                 400
   Ser His Lys Thr Arg Asn Gln Asp Glu Gly Ile Leu Pro Ser Gly Arg
                   405                 410                 415
   Arg Gly Ala Ala Arg Gly Pro Ala Gly
               420                 425

## Description

### GOVERNMENTAL INTERESTS

This invention was made with government support under R01 NS066051 and R01 NS052700 awarded by the National Institute of Health (NIH). The government has certain rights in the invention.

### FIELD OF THE INVENTION

The present disclosure relates to methods and compositions for the treatment of motor neuron degenerative disorders, and more particularly, for the treatment of amyotrophic lateral sclerosis (ALS). The methods and compositions disclosed herein employ adeno-associated virus (AAV) vectors to introduce genes into skeletal muscle that increase ciliary neurotrophic factor receptor (CNTF) and ligand expression and inhibit ALS progression.

### BACKGROUND

Amyotrophic lateral sclerosis (ALS) is an adult-onset, neurodegenerative, invariably terminal disease with death typically occurring within 2-3 years of symptom onset, and an estimated annual cost of over a billion dollars in the United States alone. There is no current cure and just one FDA approved treatment, riluzole, which prolongs survival by only 3 months.

ALS patients do not develop overt symptoms until late in the underlying disease process. Even with tertiary care centers and recent advances, diagnosis still takes approximately one year following symptom onset, further delaying treatment for all but the rare genetically diagnosed cases. Therefore, any broadly useful ALS treatment must be effective when initiated very late in the underlying disease after the ALS patient has been diagnosed. While the mechanism(s) underlying ALS are still being determined, they undoubtedly involve a series of molecular and cellular events. Interventions effective against early steps in this process would not necessarily be expected to be effective against the later steps occurring when patients are finally diagnosed. Therefore, it is not surprising that interventions developed by treating ALS mice at earlier disease stages have a history of failure in clinical trials.

Work with human ALS cell culture and model mice containing mutant copper zinc superoxide dismutase 1 (SOD1) indicates that multiple cell types, including glia, are involved in the motor neuron (MN) degeneration responsible for ALS symptoms and death. Many cellular and molecular causes have been proposed, but if a common cause is responsible for all ALS, it has yet to be established.

Endogenous neuroprotective mechanisms reduce the effects of neural insults, including neurodegenerative diseases. Ciliary neurotrophic factor receptor alpha (CNTFRα), the essential ligand binding subunit of the CNTF receptor, is expressed by both MNs and muscle. Its expression is increased in muscle by denervating nerve injury and denervating neuromuscular diseases in humans, including ALS. Evidence suggests that CNTF receptor signaling is a broadly effective endogenous neuroprotective mechanism suppressing ALS. In addition to protecting MNs during development and following perinatal nerve lesion, enhancing CNTF receptor signaling with exogenous CNTF delays disease progression and protects MN axons in several distinct models of ALS, including SOD1 mutant, pmn, and Wobbler mice, in contrast to the many potential therapies developed solely with SOD1 mutant mice.

Only two CNTF-related ALS clinical trials have been conducted with enough subjects and trial length to determine therapeutic effect. In both cases, CNTF was systemically injected into ALS patients. The systemic CNTF injections produced systemic side effects, most notably weight loss, as in CNTF injected mice. Systemic CNTF injections activate non-CNTF receptors in the liver and non-neuromuscular CNTF receptors regulating weight and produce side effects in mice at doses at least 10-fold lower than those needed to decrease disease in ALS mice. Therefore, it is understandable that the clinical trials failed in that they used systemic CNTF doses selected to produce minimal side effects, which are presumably also well below the doses needed to affect ALS in humans. Clinical trials demonstrated that ALS cannot be treated with systemic CNTF injections because side effects limit the amount of CNTF that can be administered. Clearly, there is a continuing need for ALS treatments that avoid negative systemic side effects and are effective even when initiated late in the underlying disease process.

### SUMMARY

Described herein are methods and compositions for the treatment of motor neuron degenerative disorders, particularly ALS, that do not depend on the cause of the disorder, but are instead based on the finding that enhancing an endogenous neuroprotective, anti-ALS mechanism can inhibit motor neuron degeneration and ALS resulting from a wide variety of cellular and molecular causes. Because the disclosed compositions and methods are not dependent on reversing the causes, they have the potential to treat ALS before the cause(s) are identified and to be broadly effective in the likely event that ALS results from multiple causes. The compositions and methods disclosed herein employ adeno-associated virus (AAV) gene delivery vectors to introduce ciliary neurotrophic factor receptor alpha (CNTFRα), cardiotrophin-like cytokine factor 1 (CLC), and/or cytokine receptor-like factor 1 (CLF) to skeletal muscle, thereby increasing endogenous neuroprotective mechanisms and inhibiting progression of ALS.

In one embodiment, a modified adeno-associated virus (AAV) vector packaging a recombinant AAV (rAAV)-based genome is provided for use in a method for treating a motor neuron degenerative disorder, the method comprising: administering to a subject suffering from said motor neuron degenerative disorder a modified adeno-associated virus (AAV) vector packaging a recombinant AAV (rAAV)-based genome, wherein the rAAV-based genome comprises a ciliary neurotrophic factor receptor alpha (CNTFRα) cDNA insert, and wherein the method up-regulates CNTFRα RNA expression in skeletal muscle.

In another embodiment, a pharmaceutical composition is provided, the composition comprising: a muscle-tropic modified AAV vectors, said AAV vector packaging an rAAV genome, said rAAV genome engineered to comprise: (i) a CNTFRα cDNA insert; and (ii) a promoter; and a pharmaceutically acceptable excipient.

These and other objects, features, embodiments, and advantages will become apparent to those of ordinary skill in the art from a reading of the following detailed description and the appended claims.

### BRIEF DESCRIPTION OF THE DRAWINGS

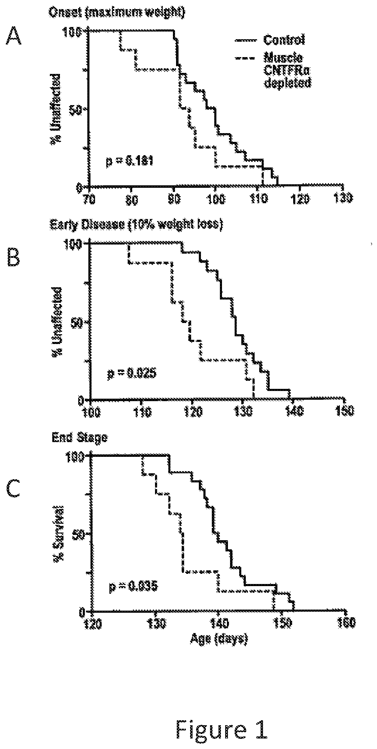

**Figure 1** is a graph showing that decreasing muscle CNTFRα accelerates SOD1^{G93A} disease. Muscle CNTFRα depleted (mlclf-Cre+ homozygous floxed CNTFRα) SOD1^{G93A} mice (n=8) and littermate SOD1^{G93A} controls (mlclf-Cre+ heterozygous or non-floxed CNTFRα; n=18) were monitored. 2-tailed log rank p values shown. Results are shown for (A) onset, (B) early disease, and (C) end stage disease. As expected, no group difference in SOD1^{G93A} copy number by qPCR; CNTFRα depleted mice = 102±2% of controls; p=0.64; t-test.

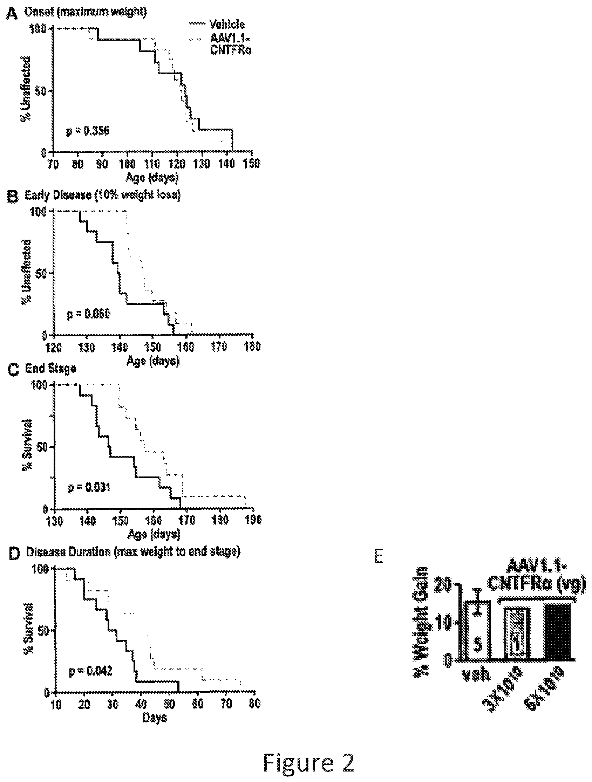

**Figure 2** is a graph showing AAV1.1-CNTFRα treatment inhibits SOD1^{G93A} disease progression. AAV1.1-CNTFRα or vehicle (PBS) was injected unilaterally into lateral gastrocnemius and soleus muscles of randomly assigned SOD1^{G93A} littermates at 120-130 days of age. Treatment delayed end stage paralysis (C) and slowed disease progression (D). Onset (A) and early disease (B) results reflect late treatment. 2-tailed log rank p values shown (vehicle, n=12; AAV1.1-CNTFRα, n=11). As shown in (E), AAV1.1-CNTFRα did not affect rate of weight gain through 15 wks post-injection. Wild type mice received vehicle (Veh), 3×10¹⁰vg or 6×10¹⁰vg of AAV1.1-CNTFRα. All male mice for weight gain pilot; n/condition in bars.

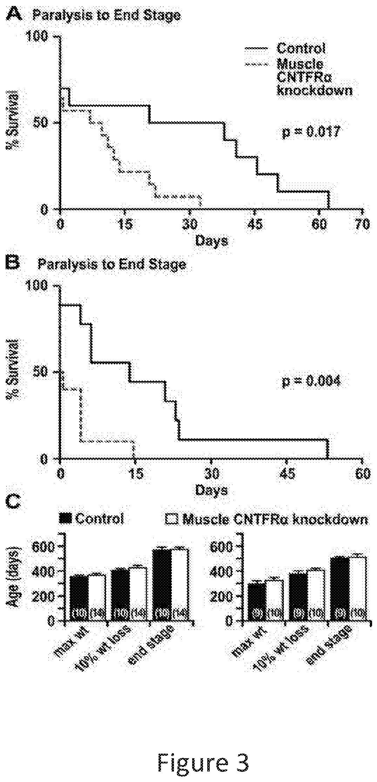

**Figure 3** is a graph showing muscle specific CNTFRα knockdown accelerates the final phase of SOD1^{G37R} disease. Muscle specific CNTFRα knockdown with mlclf-Cre (A) (n=10 control, 14 knockdown); and adult onset muscle specific CNTFRα knockdown with HSA-MCM (B) (n=9 control, 10 knockdown) both greatly accelerated the SOD1^{G37R} final paralytic phase (2-tailed log rank test p values shown), while not affecting earlier disease stages (C) mlclf-Cre (left graph) and HSA-MCM (right graph), all p>0.2; n in bars, mean ± SEM shown.

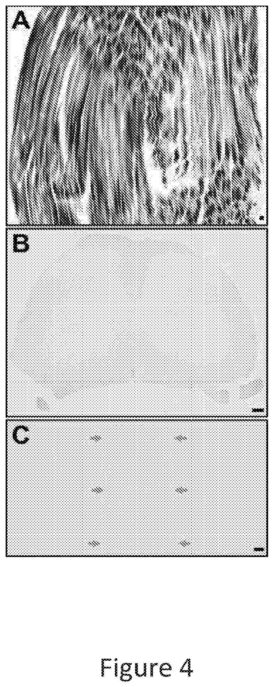

**Figure 4** shows adult induction of tamoxifen-inducible HSA-MCM leads to floxed gene excision specifically in all skeletal muscle fibers (e.g., tibialis muscle in Fig. 4(A)) with no excision in other tissues including spinal cord (Fig. 4(B)) and peripheral nerve (Fig 4(C), arrows). Fig. (A)-(C) = Xgal histology of ROSA26+ reporter tissue. Scale bars = 50µm.

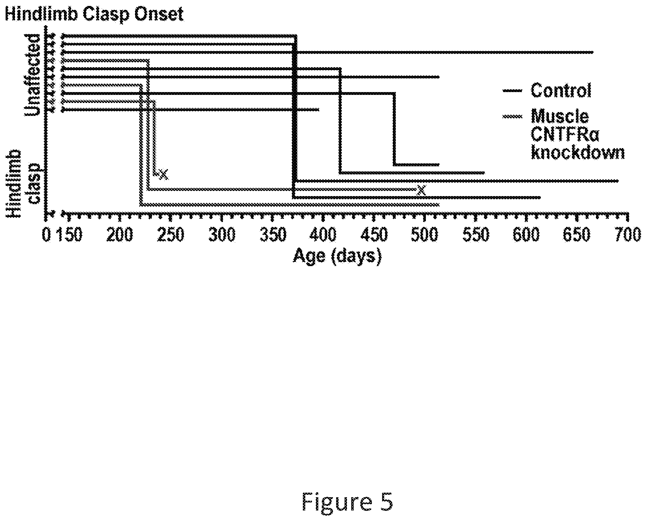

**Figure 5** shows muscle specific CNTFRα knockdown accelerates hindlimb clasp onset in TDP-43^{Q331K} disease. Adult onset muscle-specific (HSA-MCM) CNTFRα knockdown and control mice (all TDP-43^{Q331K}) were monitored 2X/wk for the TDP-43^{Q331K}- induced hindlimb clasp motor deficit (blind to genotype, as with all measures). All knockdown mice displayed the deficit significantly before any of the controls. Unlike TDP-43^{Q331K} mice without the knockdown, two knockdown mice reached end stage (X). Lines correspond to individual mice.

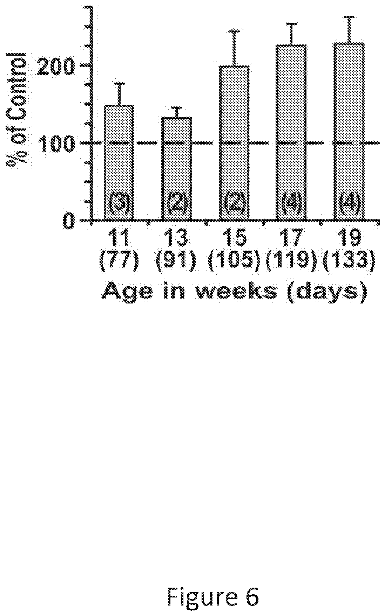

**Figure 6** shows muscle CNTFRα is induced early in SOD1^{G93A} disease. qRT-PCR of gastrocnemius CNTFRα RNA in SOD1^{G93A} mice relative to wild type littermate controls. n in bars, mean ± SEM shown.

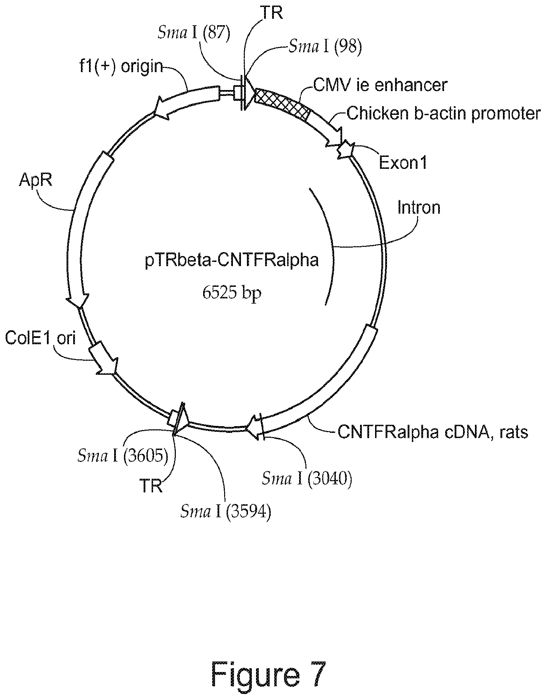

**Figure 7** is a schematic representation of a vector comprising a rat CNTFRα cDNA insert (SEQ ID NO: 1).

### DETAILED DESCRIPTION

The details of one or more embodiments of the presently-disclosed subject matter are set forth in this document. Modifications to embodiments described in this document, and other embodiments, will be evident to those of ordinary skill in the art after a study of the information provided in this document.

The practice of the present invention will employ, unless otherwise indicated, conventional methods of virology, microbiology, molecular biology and recombinant DNA techniques within the skill of the art. Such techniques are explained fully in the literature. See, e.g., Sambrook et al. Molecular Cloning: A Laboratory Manual (Current Edition); DNA Cloning: A Practical Approach, Vol. I & II (D. Glover, ed.); Oligonucleotide Synthesis (N. Gait, ed., Current Edition); Nucleic Acid Hybridization (B. Hames & S. Higgins, eds., Current Edition); Transcription and Translation (B. Hames & S. Higgins, eds., Current Edition); CRC Handbook of Parvoviruses, vol. I & II (P. Tijssen, ed.); Fundamental Virology, 2nd Edition, vol. I & II (B. N. Fields and D. M. Knipe, eds.); Freshney Culture of Animal Cells, A Manual of Basic Technique (Wiley-Liss, Third Edition); and Ausubel et al. (1991) Current Protocols in Molecular Biology (Wiley Interscience, NY).

While the following terms are believed to be well understood by one of ordinary skill in the art, definitions are set forth to facilitate explanation of the presently-disclosed subject matter. Unless defined otherwise, all technical and scientific terms used herein have the same meaning as commonly understood by one of ordinary skill in the art to which the presently-disclosed subject matter belongs.

Unless otherwise indicated, all numbers expressing quantities of ingredients, properties such as reaction conditions, and so forth used in the specification and claims are to be understood as being modified in all instances by the term "about." Accordingly, unless indicated to the contrary, the numerical parameters set forth in this specification and claims are approximations that can vary depending upon the desired properties sought to be obtained by the presently-disclosed subject matter.

As used herein, the term "about," when referring to a value or to an amount of mass, weight, time, volume, concentration or percentage is meant to encompass variations of in some embodiments ±20%, in some embodiments ±10%, in some embodiments ±5%, in some embodiments ±1%, in some embodiments ±0.5%, and in some embodiments ±0.1% from the specified amount, as such variations are appropriate to perform the disclosed method.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

As used in this specification and the appended claims, the singular forms "a," "an" and "the" include plural references unless the content clearly dictates otherwise.

"Functionally equivalent," as used herein, refers to a CNTFRα, CLC, or CLF polypeptide that retains some or all of the biological properties regarding inhibition of motor neuron degenerative disorders, such as ALS, but not necessarily to the same degree, as a native CNTFRα, CLC, or CLF molecule.

"Homology" refers to the percent similarity between two polynucleotide or two polypeptide moieties. Two polynucleotide, or two polypeptide sequences are "substantially homologous" to each other when the sequences exhibit at least about 50%, at least about 75%, at least about 80%-85%, at least about 90%, or at least about 95%-99% or more sequence similarity or sequence identity over a defined length of the molecules. As used herein, substantially homologous also refers to sequences showing complete identity to the specified polynucleotide or polypeptide sequence.

In general, "identity" refers to an exact nucleotide-to-nucleotide or amino acid-to-amino acid correspondence of two polynucleotides or polypeptide sequences, respectively. Percent identity can be determined by a direct comparison of the sequence information between two molecules by aligning the sequences, counting the exact number of matches between the two aligned sequences, dividing by the length of the shorter sequence, and multiplying the result by 100.

By "variant" is meant a biologically active derivative of the reference molecule, or a fragment of such a derivative, that retains desired activity, such as anti-ALS activity in the assays described herein. In general, the term "variant" refers to compounds having a native polypeptide sequence and structure with one or more amino acid additions, substitutions (generally conservative in nature) and/or deletions, relative to the native molecule, so long as the modifications do not destroy anti-ALS activity. Preferably, the variant has at least the same biological activity as the native molecule.

As used herein, the term "motor neuron degenerative disorder" refers to a degenerative disorder affecting a neuron with motor function. Motor neuron degenerative disorders include, but are not limited to, amyotrophic lateral sclerosis (ALS), primary lateral sclerosis (PLS), progressive muscular atrophy (PMA), progressive bulbar palsy (PBP), pseudobulbar palsy, peripheral neuropathy, spinal muscular atrophy, and Kennedy's disease. Motor neuron degenerative disorders cause increasing disability and can be terminal in nature. In a particular embodiment, the motor neuron degenerative disorder treated by the compositions and methods disclosed herein is ALS.

ALS is characterized by stages progressing in severity. In early stage ALS disease, muscles may be weak and soft or stiff, tight, and spastic. Muscle cramping and twitching occurs, as does loss of muscle bulk. Symptoms may be limited to a single body region or mild symptoms may affect more than one region. The subject suffering from ALS may experience fatigue, poor balance, slurred words, weak grip, tripping, or other minor symptoms. Middle stage ALS is characterized by more widespread symptoms, muscle paralysis, or muscle weakening. Cramping and twitching may also be present. Unused muscles may cause contractures, whereby joints may become rigid, painful, and deformed. Weakness in swallowing muscles may cause choking and difficulties eating. Weakened breathing muscles can lead to respiratory insufficiency, particularly when lying prone. Subjects may also experience inappropriate laughing or crying (pseudobulbar affect). In late stage ALS, most voluntary muscles are paralyzed. Respiratory muscles are severely compromised. Mobility is limited and assistance is required for personal care. Poor respiration may cause fatigue, confusion, headaches, and pneumonia. Speech, eating, and drinking may not be possible. In certain embodiments, the modified AAV gene delivery vectors packaging rAAV genomes comprising cDNA inserts described herein are useful in treating a subject suffering from ALS. In a specific embodiment, the modified AAV vectors are useful in treating subjects suffering from early, middle, or late stage ALS. In a very specific embodiment, the modified AAV vectors are useful in treating late stage ALS disease.

Studies have shown that a subset of patients with ALS possess mutations in certain genes. One such mutation occurs in the TDP-43 (TARDBP) gene, which suggests that gain of toxic function or loss of function in TDP-43 is an underlying cause of ALS. Pesiridis, et al., Mutations in TDP-43 link glycine-rich domain functions to amyotrophic lateral sclerosis, Human Mol. Genet. 18(R2): R156-R162 (2009). In certain embodiments, the methods and compositions disclosed herein are useful for treating ALS characterized by one or both of at least one TDP-43 mutation and abnormal TDP-43 distribution in a subject.

As used herein, the term "subject" refers to any mammalian subject, including humans.

An "effective amount" is an amount sufficient to effect beneficial or desired results. An effective amount can be administered in one or more administrations, applications or dosages. The effective amount of modified AAV vectors for use in the compositions and methods herein will vary with the motor neuron degenerative disorder being treated, the age and physical condition of the subject to be treated, the severity of the condition, the duration of the treatment, the nature of concurrent therapy, the particular modified AAV vector being employed, the particular pharmaceutically-acceptable carriers utilized, and like factors within the knowledge and expertise of the attending physician.

### Methods

The present disclosure is based on the discovery that increased CNTF receptor alpha expression in denervated muscle inhibits ALS. CNTFRα is attached to the extracellular surface of the plasma membrane by a glycosyl-phosphatidylinositol (GPI) linkage and is released following nerve lesion in soluble, functional form that may enhance CNTF receptor activity in MNs. As described herein, CNTFRα RNA expression is increased in the skeletal muscle of a wide variety of ALS model mice (as in human ALS) and selective *in vivo* genetic disruption shows that in each case this muscle CNTFRα expression can inhibit the disease. Together, these unexpected findings indicate that the increased muscle CNTFRα expression in human ALS is an endogenous neuroprotective, anti-ALS response and that enhancing this response slows disease progression and/or reverses, at least temporarily, motor symptoms associated with ALS.

As discussed herein, SOD1^{G93A} ALS mice were injected intramuscularly with a muscle tropic AAV-CNTFRα vector to increase muscle CNTFRα expression. Treatment delayed end stage paralysis and slowed disease progression, even though only a local injection was administered. Most importantly, treatment was effective when initiated later in the disease than other proposed treatments known in the art. Given the clinical reality that ALS treatments must be effective when started very late in the underlying disease, the presently disclosed methods and compositions represent a promising ALS therapeutic approach.

Targeted increase in muscle CNTFRα expression via gene therapy is less likely to produce the side effects seen with systemic CNTF injection. First, unlike CNTF, CNTFRα does not contribute to non-CNTF receptor signaling. Second, unlike CNTF injections, increasing CNTFRα expression should enhance CNTF receptor signaling only where and when endogenous CNTF receptor ligands are active because, without the ligands, increased receptor expression does not increase signaling. Third, CNTF knockout studies indicate that endogenous CNTF is not active in weight regulation, the primary side effect of exogenous CNTF administration. Fourth, unlike CNTF injections, targeted increases in muscle CNTFRα expression should enhance a natural, endogenous, ALS-induced response (i.e., the increase in muscle CNTFRα RNA). As described herein, targeting of muscle CNTFRα led to no side effects and reduced ALS associated weight loss, the opposite of the CNTF related side effect observed with systemic administration. The compositions and methods disclosed herein target muscle CNTFRα with high level, continuous changes in gene expression.

Adeno-associated virus (AAV) is a small, nonenveloped icosahedral virus that infects humans and other primates, but causes only weak immune response and is not considered pathogenic. At present, multiple AAV serotypes (AAV1-AAV13) have been sequenced and studies have identified other AAV genomes. AAVs differ in tropism for target tissues, including cardiac and skeletal muscle, liver and lung tissue, and cells in the CNS. Differing tropisms can be exploited for use in gene therapy, enabling the directed treatment of specific tissues. Drouin, et al., Adeno-associated virus structural biology as a tool in vector development, Future. Virol. 8(12): 1183-99 (2013). AAV vectors modified to be muscle-tropic are particularly useful in gene therapy treatments for motor neuron degenerative disorders. By an "AAV vector" is meant a vector derived from an adeno-associated virus serotype.

The AAV vectors disclosed herein are engineered to package a recombinant AAV-based genome (rAAV). rAAV genomes can be single stranded rAAV, which requires synthesis of a complementary DNA strand, or self-complementary rAAV (scrAAV), which packages two shorter DNA strands that are complementary to each other. By avoiding second-strand synthesis, scrAAV can express more quickly, although vector capacity is reduced. In certain embodiments, the AAV vectors disclosed herein comprise an rAAV genome comprising single stranded rAAV, self-complementary rAAV (scrAAV), and combinations thereof.

Ciliary neurotrophic factor receptor alpha (CNTFRα) is an essential ligand binding subunit of the CNTF receptor, which is composed of CNTFRα, a leukemia inhibitory factor receptor β (LIFRβ), and glycoprotein (gp) 130. While LIFRβ and gp130 are found in other related receptors, CNTFRα is unique to CNTF receptors and is required for all known forms of CNTF receptor signaling. A number of CNTFRα polynucleotide and amino acid sequences are known. The degree of homology between rat, human, and mouse proteins is about 94%. Representative protein coding sequences of rat, human, and mouse CNTFRα are set forth herein as SEQ ID NOs: 2, 3, and 4, respectively. Additional CNTFRα sequences are known in the art. See, e.g., NCBI accession numbers AL160270, AL537375, BM714356, BT019824, BX364434, BC001492, BI829871, and BM714356 (human); BC046974, CX202454, AL831723, and BY715214 (mouse); S54212 (rat); JU332908 (rhesus monkey); and AF529215 (dog) (all accession numbers accessed March 31, 2017, 12:00 a.m. EST); as well as other known mammalian CNTFRα sequences. Any of these sequences, as well as variants thereof, such as sequences substantially homologous and functionally equivalent to these sequences, will find use in the present methods. In embodiments according to the invention, the modified AAV vector comprises an rAAV genome comprising a CNTFRα cDNA insert, wherein in some embodiments the vector produces a protein selected from SEQ ID NOs: 2, 3, or 4, or a substantially homologous or functionally equivalent variant thereof.

Cardiotrophin-like cytokine factor 1 (CLC) is a member of the gp130 cytokine family and encodes cardiotrophin-like cytokine factor 1. CLC forms a heterodimer complex with cytokine receptor-like factor 1 (CLF). This dimer competes with ciliary neurotrophic factor (CNTF) for binding to the ciliary neurotrophic factor receptor (CNTFR) complex, and activates the Jak-STAT signaling cascade. CLC can be actively secreted from cells by forming a complex with soluble type I CLF or soluble CNTFR. CLC is a potent neurotrophic factor, B-cell stimulatory agent and neuroendocrine modulator of pituitary corticotroph function. A number of CLC polynucleotide and amino acid sequences are known. Representative protein coding sequences of human and mouse CLC are set forth herein as SEQ ID NOs: 5 and 6, respectively. Additional CLC sequences are known in the art. See, e.g., NCBI accession numbers AK298052, BC012939, DC393345, and BM846622 (human); AC109138, AI451696, AK137396, BB786146, BC104258, and CX202966 (mouse); and BC098643 (rat) (all accession numbers accessed March 31, 2017, 12:00 a.m. EST); as well as other mammalian CLC sequences. Any of these sequences, as well as variants thereof, such as sequences substantially homologous and functionally equivalent to these sequences, will find use in the present methods. In some embodiments, a modified AAV vector comprises an rAAV genome comprising a CLC cDNA insert, wherein the vector produces a protein selected from SEQ ID NOs: 5 or 6, or a substantially homologous or functionally equivalent variant thereof.

Cytokine receptor-like factor 1 (CLF) encodes a member of the cytokine type I receptor family. The protein forms a secreted complex with cardiotrophin-like cytokine factor 1 (CLC) and acts on cells expressing ciliary neurotrophic factor receptors. The complex can promote survival of neuronal cells. A number of CLF polynucleotide and amino acid sequences are known. Representative protein coding sequences of human and mouse CLF are set forth herein as SEQ ID NOs: 7 and 8, respectively. Additional CLF sequences are known in the art. See, e.g., NCBI accession numbers AF073515 and AY358291 (human); AC157774 (mouse); CH474031 (rat); and BC076526 (zebrafish) (all accession numbers accessed March 31, 2017, 12:00 a.m. EST); as well as other mammalian CLF sequences. Any of these sequences, as well as variants thereof, such as sequences substantially homologous and functionally equivalent to these sequences, will find use in the present methods. In some embodiments, a modified AAV vector comprises an rAAV genome comprising a CLF cDNA insert, wherein the vector produces a protein selected from SEQ ID NOs: 7 or 8, or a substantially homologous or functionally equivalent variant thereof.

Each rAAV genome is engineered to comprise at least one cDNA insert protein coding sequence. In embodiments, the cDNA insert is a ciliary neutrophic factor receptor alpha (CNTFRα) cDNA insert. In embodiments, a cDNA insert may be a cardiotrophin-like cytokine factor 1 (CLC) cDNA insert, and/or a cytokine receptor-like factor 1 (CLF) cDNA insert. While not desiring to be bound by theory, it is believed that the administration of AAV vectors packaging rAAV genomes comprising CLC, and/or CLF cDNA inserts to a subject suffering from a motor neuron degenerative disorder will enhance the therapeutic effects of the administration of AAV vectors packaging rAAV CNTFRα genomes by providing ligands for the increased receptor expression. Moreover, it is believed that the administration of AAV vectors packaging rAAV genomes comprising CLC and/or CLF cDNA inserts to a subject suffering from a motor neuron degenerative disorder will enhance the therapeutic effects of the increase in endogenous muscle CNTFRα.

The AAV vectors disclosed herein comprise control elements capable of directing the *in vivo* transcription and translation of CNTFRα, CLC, or CLF. In certain embodiments, the control elements comprise a promoter. The term "promoter" is used herein to refer to a nucleotide region comprising a DNA regulatory sequence, wherein the regulatory sequence is derived from a gene which is capable of binding RNA polymerase and initiating transcription of a downstream (3'-direction) coding sequence. In one embodiment, the promoter is selected from a cytomegalovirus early enhancer element/chicken beta-actin (CAG) promoter and a muscle-specific promoter. In a specific embodiment, the muscle-specific promoter is a muscle specific creatine kinase (MCK) promoter. In a more specific embodiment, the MCK promoter comprises double muscle specific creatine kinase (dMCK) or triple muscle specific creatine kinase (tMCK) promoter. The skilled artisan will appreciate that other promoters are known in the art and suitable for use in controlling and directing the *in vivo* transcription and translation of CNTFRα, CLC, or CLF.

In a particular embodiment, combinations of modified AAV vectors are administered to a subject. For example, in one embodiment, a modified AAV vector packaging an rAAV-based genome comprising a CNTFRα cDNA insert is provided for use in a method for treating a motor neuron disorder in a subject suffering from a motor neuron disease, the method comprising administering (1) the modified AAV vector packaging an rAAV-based genome comprising a CNTFRα cDNA insert, together with (2) a modified AAV vector packaging an rAAV-based genome comprising a CLC cDNA insert. In another embodiment, the method comprises administering (1) the modified AAV vector packaging an rAAV-based genome comprising a CNTFRα cDNA insert, together with (2) a modified AAV vector packaging an rAAV-based genome comprising a CLF cDNA insert. In another embodiment, the method comprises administering (1) the modified AAV vector packaging an rAAV-based genome comprising a CNTFRα cDNA insert, together with (2) a modified AAV vector packaging an rAAV-based genome comprising a CLC cDNA insert, and (3) a modified AAV vector packaging an rAAV-based genome comprising a CLF cDNA insert. In some described arrangements, the method comprises administering (1) a modified AAV vector packaging an rAAV-based genome comprising a CLC cDNA insert, together with (2) a modified AAV vector packaging an rAAV-based genome comprising a CLF cDNA insert.

Modified AAV vectors comprising distinct cDNA inserts can be co-administered or sequentially administered. When sequentially administered, the duration of time between administering a first AAV vector and administering a subsequent AAV vector may be from about 1 hour, 2 hours, 3 hours, 4 hours, 6 hours, 12 hours, 24 hours, one week, two weeks, three weeks, 4 weeks, one month, up to six months. In some embodiments, administration of one or more AAV vectors as described herein is initiated prior to, contemporaneous with, or even after onset of motor symptoms in the subject. In a specific embodiment, administration is initiated during late stage ALS disease and is effective to slow disease progression and/or at least temporarily partially reverse motor symptoms in the subject, including paralysis.

Administration of modified AAV vectors comprises systemic and non-systemic administration. In particular embodiments, non-systemic administration comprises intramuscular (IM) injection to one or more muscles selected from non-respiratory skeletal muscles, respiratory skeletal muscles, and combinations thereof. In certain embodiments, IM injections are administered unilaterally to a subject. In other embodiments, IM injections are administered bilaterally to the subject, for example, bilaterally to the gastrocnemius muscle on each side of the subject. When two or more AAV vectors packaging genomes comprising distinct cDNA inserts are administered, the two or more AAV vectors can be independently administered; for example, one AAV vector may be administered systemically, and a second AAV vector may be administered non-systemically. Similarly, the location (i.e., skeletal muscle) and manner (i.e., unilateral or bilateral) of injection for non-systemic administration may vary between distinct AAV vectors.

### Pharmaceutical Compositions

Pharmaceutical compositions will comprise sufficient genetic material to produce a therapeutically effective amount of the CNTFRα, and optionally the CLC, and/or CLF of interest, i.e., an amount sufficient to reduce or ameliorate symptoms of the disease state in question or an amount sufficient to confer the desired benefit. The compositions will also contain a pharmaceutically acceptable excipient. Such excipients include any pharmaceutical agent that does not itself induce the production of antibodies harmful to the individual receiving the composition, and which may be administered without undue toxicity. Pharmaceutically acceptable excipients include, but are not limited to, sorbitol, any of the various TWEEN compounds, and liquids such as water, saline, glycerol and ethanol. Pharmaceutically acceptable salts can be included therein, for example, mineral acid salts such as hydrochlorides, hydrobromides, phosphates, sulfates, and the like; and the salts of organic acids such as acetates, propionates, malonates, benzoates, and the like. Additionally, auxiliary substances, such as wetting or emulsifying agents, pH buffering substances, and the like, may be present in such vehicles. A thorough discussion of pharmaceutically acceptable excipients is available in REMINGTON'S PHARMACEUTICAL SCIENCES (Mack Pub. Co., N.J. 1991).

One particularly useful formulation comprises recombinant AAV vectors in combination with one or more dihydric or polyhydric alcohols, and, optionally, a detergent, such as a sorbitan ester. See, for example, International Publication No. WO 00/32233.

Pharmaceutical compositions described herein may be formulated for intramuscular administration or intravenous administration and may be administered according to any of the methods disclosed herein.

As is apparent to those skilled in the art in view of the teachings of this specification, an effective amount of viral vector which must be added can be empirically determined. Representative doses are detailed below. Administration can be effected in one dose, continuously or intermittently throughout the course of treatment. Methods of determining the most effective means and dosages of administration are well known to those of skill in the art and will vary with the viral vector, the composition of the therapy, the target cells, and the subject being treated. Single and multiple administrations can be carried out with the dose level and pattern being selected by the treating physician. In certain embodiments, increasing a dose level increases the therapeutic benefit of the composition.

It should be understood that distinct AAV vectors, each expressing one or more different transgenes, can also be delivered as described herein. Furthermore, it is also intended that the viral vectors delivered by the methods of the present invention be combined with other suitable compositions and therapies. Where the transgene is under the control of an inducible promoter, certain systemically delivered compounds such as muristerone, ponasteron, tetracyline or aufin may be administered in order to regulate expression of the transgene.

Modified AAV vectors may be delivered directly to muscle by injection with a needle, catheter, or related device, using techniques known in the art. For *in vivo* delivery, the AAV vectors will be formulated into pharmaceutical compositions and one or more dosages may be administered directly in the indicated manner. A therapeutically effective dose will include on the order of from about 10⁸/kg to 10¹⁶/kg of the AAV vectors, optionally from about 10¹⁰/kg to 10¹⁴/kg, and optionally from about 10¹¹/kg to 10¹³/kg of each modified AAV vector (or viral genomes, also termed "vg"), or any value within these ranges.

In one embodiment, a pharmaceutical composition for the treatment of a motor neuron degenerative disorder is provided, the composition comprising: one or more muscle-tropic modified AAV vectors, each of said AAV vectors packaging an rAAV genome, each of said rAAV genomes engineered to comprise: (i) a cDNA insert selected from the group consisting of a CNTFRα cDNA insert, a CLC cDNA insert, and a CLF cDNA insert; and (ii) a promoter; and a pharmaceutically acceptable excipient. In certain embodiments, the rAAV genome comprises single-stranded rAAV, self-complementary rAAV (scrAAV), or combinations thereof. As described above, in some embodiments the promoter is selected from the group consisting of a cytomegalovirus early enhancer element/chicken beta-actin (CAG) promoter and a muscle-specific promoter. In a specific embodiment, the promoter is a muscle specific promoter selected from the group consisting of muscle specific creatine kinase (MCK) promoter, double MCK (dMCK) promoter, and triple MCK (tMCK) promoter.

### Examples

The following examples are given by way of illustration and are in no way intended to limit the scope of the present invention.

### Example 1

### General Procedures

Mice were monitored for weight and inability to right themselves in 30 sec when placed on side, i.e., -universal index of end stage paralysis (loss of motor function) in ALS mouse models. These data, and age at max weight, age at 10% weight loss from max, and time from max weight to end stage (widely used indexes of disease onset, early disease and disease duration, respectively) were analyzed by 2-tailed log rank test.

### Example 2

### Effects of CNTFRα Gene Disruption

The CNTF receptor contains CNTFRα, leukemia inhibitory factor receptor β and gp130. Only CNTFRα is unique to the CNTF receptor, absolutely required for all CNTF receptor signaling, and not involved in any other signaling. Therefore, disruption of CNTFRα *in vivo* is the best way to determine the endogenous roles played by CNTF receptor signaling. Neuromuscular CNTF receptors are restricted to MNs and muscle.

Human and animal data suggest that MN terminal loss is the initial critical step in MN degeneration leading to ALS symptoms. When the CNTFRα gene is selectively disrupted in adult MNs (confirmed by CNTFRα immunohistochemistry) by crossing floxed CNTFRα mice with "SLICK-A" mice and treating adults with tamoxifen to drive Cre activity and a YFP reporter in a subset of MNs (but not muscle), it has no effect on MN terminals (homozygous floxed mice had 97.9% as many reporter+ terminals as littermate controls; p>0.5, t=0.02, N=6 pair). Therefore, without insult, adult MN CNTFRα expression is not required to maintain terminals. However, when the "CreER" construct and a YFP reporter was used in an analogous experiment to disrupt the CNTFRα gene in both a subset of MNs, comparable to SLICK-A, and in muscle (both confirmed) the depleted mice contained only 28.9% as many reporter+ terminals as their littermate controls (p=0.005; t= 5.53; N=5 pair). Therefore, the additional loss of muscle CNTFRα expression led to terminal loss not seen with MN CNTFRα disruption alone, indicating that muscle CNTFRα expression can surprisingly maintain MN terminals under some circumstances (in MNs lacking CNTFRα).

The above data indicating that muscle CNTFRα expression helps maintain MN terminals also support that it can inhibit ALS, consistent with the increased muscle CNTFRα expression in denervating human disease, including ALS. Mlclf-Cre was then used to selectively deplete muscle CNTFRα expression in the most commonly used ALS model, "high copy number" SOD1^{G93A} mice (Jax. Lab. stock #004435). This led to earlier end stage paralysis (Fig. 1). Therefore, endogenous muscle CNTFRα expression reduces the effects of the ALS-inducing mutation. Moreover, the data likely significantly underestimate the impact of muscle CNTFRα since mlclf-Cre-induced CNTFRα depletion leaves 10-20% of muscle CNTFRα expression intact. Regardless, the data indicate that the increased muscle CNTFRα expression in human ALS similarly reduces the effects of human ALS and that further enhancing muscle CNTFRα expression may further inhibit ALS.

### Example 3

### Enhancing muscle CNTFRα expression slows SOD1^{G93A} disease progression even when initiated very late in the disease

Of the potential ALS treatments developed with SOD1 mutant mice and taken to human trials only riluzole had any clinical benefit. Riluzole modestly slowed both human and mouse disease progression. All other treatments failed to slow mouse or human disease progression, while only delaying mouse disease onset, prompting the conclusion by leaders in the field that recognizing that success at human trial will require slowing of disease progression, the SOD1-mutant mice have perfectly predicted the success of riluzole and the failure of efficacy of each other drug attempted in human trial. Therefore, despite the examples of failed ALS treatments developed with SOD1-mutant mice, the assay is completely valid when used correctly to measure changes in disease progression.

Moreover, it is not enough that treatments slow disease progression. Late ALS symptom onset and subsequent, long delays in clinical diagnosis before treatment initiation dictate that any broadly useful ALS treatment must be effective when initiated very late in the underlying disease.

Safety and long term expression profiles of AAV have made it the vector of choice for human gene therapy. AAV1 is a preferred AAV capsid for intramuscular (IM) injection. AAV1.1 is a Samulski lab, AAV1-derived capsid engineered to direct enhanced skeletal muscle expression. AAV1.1 was used to package an AAV2-based vector (Figure 7, SEQ ID NO: 1, gift from Dr. S. Zolotukhin; Univ. of FL) containing a CMV enhancer/chicken beta-actin (CBA) promoter and a rat CNTFRα cDNA.

Fig. 2 shows AAV1.1-CNTFRα treatment inhibits SOD1^{G93A} disease progression. AAV1.1-CNTFRα or vehicle (PBS) was injected unilaterally into lateral gastrocnemius and soleus muscles of randomly assigned SOD1^{G93A} littermates at 120-130 days of age (late in disease progression, 81-88% of median control end stage age; well after substantial MN terminal loss, which starts at -50 days of age). Injections included 3×10¹⁰vg of AAV1.1-CNTFRα prep frozen and thawed only once between generation and use (n=3) or 6×10¹⁰vg of AAV1.1-CNTFRα prep frozen and thawed twice (n=8) (no prep difference [163.1±3.2 vs 160.3±4.6 days to end stage]; combined AAV1.1-CNTFRα results shown). Wild type mice received vehicle (Veh), 3×10¹⁰vg or 6×10¹⁰vg of AAV1.1-CNTFRα. All male mice for weight gain pilot; n/condition in bars. Treatment delayed median end stage paralysis by 11 days (AAV1.1-CNTFRα, 158 days; controls, 147 days; Fig. 2C; no hindlimb difference in time to paralysis), even though it was initiated only 10-20 days before the first vehicle mice reached end stage paralysis (Fig. 2(C)) and slowed median disease progression by 37% (AAV1.1-CNTFRα, 41 days; controls, 30 days; Fig. 2(D)), i.e., increased disease duration per standard definition of time from max weight to end stage paralysis).

The onset (A) and early disease (B) results are expected given the late treatment. 2-tailed log rank p values shown (vehicle, n=12; AAV1.1-CNTFRα, n=11). The later disease in controls in Fig. 2 vs. Fig. 1 reflects the mixed B6/SvEvBrd (Fig. 1) vs. pure B6 (Fig. 2) backgrounds. Control values in Fig. 2 are consistent with previous pure B6 SOD1^{G93A} studies. As expected, no difference in SOD1^{G93A} gene copy number; AAV1.1-CNTFRα mice = 94±9% of controls; p=0.57; t-test. As shown in Fig. 2(E), in contrast to the weight loss seen with systemic CNTF injection in humans and mice, AAV1.1-CNTFRα did not affect rate of weight gain through 15 wks post-injection, which should also detect any other significant side effects.

Of the many reports of SOD1^{G93A} ALS mouse treatments, no other treatment to date has decreased SOD1^{G93A} disease when initiated as late as the AAV1.1-CNTFRα treatment, with most treatments initiated much earlier. As expected, there are several reports of earlier treatment producing large therapeutic effects that diminish or disappear when treatment is started later (but still earlier than the instant experimental results). Given the clinical reality that ALS treatments need to be effective when started very late in the underlying disease, the AAV1.1-CNTFRα treatment is arguably the most promising ALS therapeutic proposed to date.

Unlike the weight loss side effect seen with systemic CNTF administration, the AAV1.1-CNTFRα surprisingly had the opposite effect, delaying weight loss in SOD1^{G93A} mice (Fig. 2(B)), as expected from the delay in disease progression. Moreover, injection of wild type mice with AAV1.1-CNTFRα had no effect on weight or even rate of weight gain (Fig. 2(E)). This is a sensitive global screen for the first sign of side effects since a CNTF dose producing sustained weight loss (in a human weight loss trial) produced no other significant side effects (more patients dropped out of the vehicle group due to "adverse events" than dropped out of the CNTF group). This screening for any decrease in rate of weight gain, rather than just weight loss, is even more sensitive and should detect any unexpected effects on health in general.

Blind scoring of the SOD1^{G93A} mice indicated that two mice (both AAV1.1-CNTFRα injected) temporarily "recovered," with a dramatic decrease in paralysis (large decrease in righting time relative to previous measurements) for several days nearing end stage. Having lost 38.4±3.0% of their max weight, these two mice and one other AAV1.1-CNTFRα mouse gained a surprising and unexpected 23.6±2.2% of their total weight over the same 3-5 day "recovery" period before again declining. This is a unique and unprecedented outcome in the 30+ control SOD1^{G93A} mice observed. These promising data support the conclusion that enhancing muscle CNTFRα expression, even very late in the disease, may delay decline as well as temporarily reverse the disease effects.

### Example 4

### Endogenous Muscle CNTFRα Inhibits Late Stage SOD1^{G37R} Disease

Mlclf-Cre and floxed CNTFRα mice were used to specifically reduce muscle CNTFRα in SOD1^{G37R} mice, a widely used ALS model with a different human ALS-inducing mutation and lower mutant SOD1 levels, more like human ALS.

Muscle specific CNTFRα knockdown with mlclf-Cre (Fig. 3(A)) (n=10 control, 14 knockdown); and adult onset muscle specific CNTFRα knockdown with HSA-MCM (Fig. 3(B)) (n=9 control, 10 knockdown) both greatly accelerated the SOD1^{G37R} final paralytic phase (2-tailed log rank test p values shown), while not affecting earlier disease stages (Fig. 3(C); mlclf-Cre on left and HSA-MCM on right, all p>0.2; n in bars, mean ± SEM shown). All mice displayed paralysis starting with a hindlimb. Mice were monitored 2 times/wk until onset of paralysis and daily thereafter. Mice scored as "0 days" progressed from onset of paralysis to end stage within the ½ wk interval between measurements. The experiments were run on different genetic backgrounds; mlc-Cre experiment on B6/SvEvBrd, HSA-MCM experiment on B6/SvEvBrd/C3H (HSA-MCM obtained on B6/C3H) likely accounting for the somewhat different control time courses

CNTFRα knockdown specifically and greatly accelerated progression through the final disease phase (Fig. 3(A)) from onset of complete paralysis of the first hindlimb (blindly scored) to end stage (failure to right from both sides, involving hindlimbs, forelimbs, and potentially respiratory muscle). This phase (neurological score 3 to end stage) is analogous to late stage human ALS when patients have been diagnosed and are desperate for treatment inhibiting disease progression. The data suggest muscle CNTFRα profoundly inhibits ALS progression at a time when patients are treatable. The 64% decrease in this late phase (controls: 26.3±7.8 days; knockdown: 9.4±2.8 days; p=0.017) did not significantly affect the 567±25.9 day full lifespan (Fig. 3(C)), just as large clinically significant change specific to the 1-2 year final phase of human ALS would not be detected as an overall change in lifespan without many more subjects.

Muscle CNTFRα was then knocked down starting in adulthood with HSA-MCM, which drives muscle specific, tamoxifen inducible Cre, with no pre-tamoxifen leakage. Fig. 4 shows adult induction of tamoxifen-inducible HSA-MCM leads to floxed gene excision specifically in all skeletal muscle fibers (e.g., tibialis muscle in Fig. 4(A)) with no excision in other tissues including spinal cord (Fig. 4(B)) and peripheral nerve (Fig 4(C), arrows). Fig. (A)-(C) = Xgal histology of ROSA26+ reporter tissue. Scale bars = 50µm. Results confirmed CNTFRα knockdown (floxed mice muscle CNTFRα RNA reduced to 9.3±3.0% of identically treated non-floxed littermates by 1 month post-tamoxifen; n=2). This knockdown also specifically and dramatically accelerated the final disease phase (controls: 16.9±5.4 days; knockdown: 2.8±1.5 days, p=0.004; Fig. 3(B)). Results show knockdown mice progress about six times faster than controls, indicating that adult muscle CNTFRα (the clinical target) inhibits the disease.

### Example 5

### Endogenous Muscle CNTFRα Inhibits TDP-43^{Q331K} Disease

Transactivating Response Region DNA binding protein 43 (TDP-43) mutations underlie 1-5% of familial ALS. TDP-43+ aggregates are found in all sporadic ALS, suggesting that TND-43 pathology underlies most ALS disease. Unlike other available TDP-43 mouse models that display unworkable phenotype variability and/or degenerate before adulthood, a line with a human ALS-causing TDP-43^{Q331K} mutation, displays an adult-onset progressive motor deficit as needed for adult-onset disruption studies.

As shown in Fig. 5, adult onset muscle-specific (HSA-MCM) CNTFRα knockdown and control mice (all TDP-43^{Q331K}) were monitored 2 times/wk for the TDP-43^{Q331K}-induced hindlimb clasp motor deficit (blind to genotype, as with all measures). Although the TDP-43^{Q331K} deficit occurs later on this B6N/B6/C3H/SvEvBrd background than on the original B6N, the knockdown clearly accelerated its onset, in that all knockdown mice displayed the deficit significantly before any of the controls. Unlike TDP-43^{Q331K} mice without the knockdown, two knockdown mice reached end stage (Xs in Fig. 5). Lines correspond to individual mice, depicting hindlimb clasp history and age. Both sexes represented in each group with control and knockdown sex-matched littermates compared in each case. Oldest mice are on the same mixed background and come from earlier round breeding required to combine genes.

This data with HSA-MCM clearly indicate that adult onset muscle specific CNTFRα knockdown greatly accelerates TDP-43^{Q331K} disease since all three knockdown mice displayed the hindlimb clasp motor deficit long before any of the 7 controls. The data suggest an overall difference >150 days (Fig. 5). Even if all controls not yet displaying hindlimb clasp did so at the next monitoring, 2-tailed log rank analysis still indicates a highly significant muscle CNTFRα knockdown effect (p=0.0008). The TDP-43^{Q331K} CNTFRα knockdown mice also display large rotarod deficits (73.8±14.5% decrease; p=0.036) and hindlimb grip strength deficits (79.6±13.8% decrease; p=0.029) relative to age and sex matched TDP-43^{Q331K} controls. As with other neurological measures, muscle CNTFRα knockdown does not produce hindlimb clasp, grip strength, or rotarod deficits in naive mice (confirmed on this experiment's mixed background). Therefore, the data indicate the knockdown accelerates TDP-43^{Q331K} disease and muscle CNTFRα protects against this ALS-inducing mutation. Moreover, 2 of the 3 knockdown mice reached end stage: one mouse reached end stage paralysis (~250 days) and one developed intense tremors and 30% weight loss followed by death (~500 days). (Fig. 5). End stage disease is not otherwise seen (i.e., without CNTFRα knockdown) at any age in TDP-43^{Q331K} mice, indicating that muscle CNTFRα not only greatly slows disease progression but also qualitatively reduces the maximum deficit.

### Example 6

### Muscle CNTFRα Induction in ALS Mice

The data suggest that CNTFRα is induced to play the anti-ALS roles revealed by the presently disclosed knockdown data. The smaller CNTFRα knockdown effect throughout SOD1^{G93A} disease compared to the larger effect specific to final stage SOD1^{G37R} disease (Fig. 3) is consistent with the modest CNTFRα increase (~2 fold) throughout SOD1^{G93A} disease (Fig. 6) and the larger (~8 fold) increase at final stage SOD1^{G37R} disease (780±60% of controls, p=0.006, n=3) but not earlier at 1 year (96.3±1.0% of controls, p>0.8. n=4). Similarly, muscle CNTFRα RNA increases (~4.5 fold) in TDP-43^{Q331K} mice at 1 year (440±60% of controls, p=0.003, n=5) along with the knockdown effect on TDP-43^{Q331K} disease (Fig. 5), but is unchanged at 3 months before the knockdown effect (107.0±37% of controls, p>0.5, n=2).

This demonstration of *in vivo* anti-ALS activity in multiple SOD1 models and a mammalian TDP-43 model is surprising. It suggests the muscle CNTFRα increase in human ALS and all ALS models is a broadly effective endogenous anti-ALS response and that further increasing this expression therapeutically enhances anti-ALS response.

### Example 7

### Treating ALS by Increasing Muscle CNTFRα RNA and Muscle CLC RNA Together

When CLC and CNTFRα are heterologously expressed together *in vitro,* a soluble CLC/ CNTFRα complex is released, which can activate CNTF receptors and promote survival of cultured embryonic motor neurons (MNs). *In situ* hybridization indicates *in vivo* embryonic muscle cells express this combination of CLC and CNTFRα, indicating CLC/CNTFRa released from these cells protects MNs from development associated death *in vivo.* In agreement, knockout of either CLC or CNTFRα (but not CNTF) leads to embryonic MN loss. Together, the data indicate that the anti-ALS effects of endogenous muscle CNTFRα similarly involve muscle CLC/CNTFRa release protecting adult ALS MNs, either by acting on MN CNTF receptors or eliciting the release of neuroprotective muscle factors by acting on muscle CNTF receptors.

Moreover, like muscle CNTFRα RNA, muscle CLC RNA: 1) increased starting early in SOD1^{G93A} disease (238±31% of controls; p=0.0059; n=8), 2) increased in TDP-43^{Q331K} mice at 1 year (undetectable in controls and ~2 fold above detection threshold in TDP-43^{Q331K} mice; n=5) while unaffected at 3 months, and 3) selectively increased 6.9±1.5 fold; n=3 in final paralytic stage SOD1^{G37R} mice. Therefore, while not desiring to be bound by theory, the data indicate that the endogenous anti-ALS mechanism may involve simultaneous CLC and CNTFRα increases, leading to increased MN protective CLC/CNTFRa release, such that the AAV1.1-CNTFRα therapeutic effect observed derives from a further increase in muscle CLC/CNTFRa release that should, at high enough AAV1.1-CNTFRα dose, be ultimately limited by available endogenous muscle CLC. Accordingly, an AAV-induced increase in muscle CLC enhances the efficacy of AAV1.1-CNTFRα. Moreover, by selectively targeting the cell type endogenously expressing CLC and CNTFRα (i.e., muscle), it is possible to exploit the endogenous mechanisms regulating release and avoid/reduce side effects.

### Example 8

### Treating ALS by Increasing Muscle CNTFRα RNA and Muscle CLF RNA Together

CNTF receptors are also activated by a complex of CLC and CLF. CLF is also expressed in muscle and, like CLC and CNTFRα, muscle CLF expression is increased in ALS mice. Therefore, a combined increase in both muscle CNTFRα RNA and muscle CLF RNA enhances CNTF receptor activity and thereby produces a similar ALS therapeutic effect to that seen with increasing muscle CNTFRα.

### Example 9

### Treating ALS by Increasing Muscle CLC and Muscle CLF Together

CNTF receptors are also activated by a complex of CLC and CLF. CLF is also expressed in muscle and, like CLC and CNTFRα, muscle CLF expression is increased in ALS mice. Therefore, a combined increase in both muscle CLC RNA and muscle CLF RNA enhances CNTF receptor activity and thereby produces a similar ALS therapeutic effect to that seen with increasing muscle CNTFRα.

### Example 10

### Treating ALS by Increasing Muscle CNTFRα RNA, Muscle CLC RNA,

### and Muscle CLF RNA Together

Increasing expression of all three of these critical CNTF receptor signaling genes (CNTFRα, CLC, and CLF) in muscle is also therapeutic in treating ALS.

While particular embodiments of the present invention have been illustrated and described, it would be obvious to one skilled in the art that various other changes and modifications can be made without departing from the scope of the invention. The scope of the invention is defined by the appended claims.

## Claims

1. A modified adeno-associated virus (AAV) vector packaging a recombinant AAV (rAAV)-based genome, for use in a method for treating a motor neuron degenerative disorder, the method comprising: administering to a subject suffering from said motor neuron degenerative disorder a modified adeno-associated virus (AAV) vector packaging a recombinant AAV (rAAV)-based genome, wherein the rAAV-based genome comprises a ciliary neurotrophic factor receptor alpha (CNTFRα) cDNA insert, and wherein the method up-regulates CNTFRα RNA expression in skeletal muscle.

2. The modified adeno-associated virus (AAV) vector for use according to claim 1, wherein the method further comprises administering to the subject an AAV vector comprising an rAAV-based genome comprising a CLC cDNA insert, and/or wherein the method further comprises administering to the subject an AAV vector comprising an rAAV-based genome comprising a CLF cDNA insert.

3. The modified adeno-associated virus (AAV) vector for use according to claim 1, wherein the disorder comprises Amyotrophic Lateral Sclerosis (ALS).

4. The modified adeno-associated virus (AAV) vector for use according to claim 3, wherein ALS comprises late-stage ALS; or wherein the ALS is **characterized by** one or both of: at least one TDP-43 mutation; and an abnormal cellular TDP-43 distribution.

5. The modified adeno-associated virus (AAV) vector for use according to claim 1, wherein the rAAV-based genome is modified to be muscle-tropic.

6. The modified adeno-associated virus (AAV) vector for use according to claim 1, wherein the method comprises:
- administering the modified adeno-associated virus (AAV) vector to the subject by non-systemic administration; or
- administering the modified adeno-associated virus (AAV) vector to the subject by non-systemic administration and non-systemic administration comprises intramuscular (IM) administration to one or more muscles selected from non-respiratory skeletal muscles, respiratory muscles, and combinations thereof.

7. The modified adeno-associated virus (AAV) vector for use according to claim 1, wherein the method comprises initiating administration of the modified adeno-associated virus (AAV) vector to the subject prior to, contemporaneous with, or after an onset of motor symptoms in the subject.

8. The modified adeno-associated virus (AAV) vector for use according to claim 1, wherein the rAAV genome comprises a promoter selected from a cytomegalovirus early enhancer element/chicken beta-actin (CAG) promoter and a muscle-specific promoter.

9. The modified adeno-associated virus (AAV) vector for use according to claim 8, wherein the promoter comprises a muscle-specific promoter comprising a muscle specific creatine kinase (MCK) promoter.

10. The modified adeno-associated virus (AAV) vector for use according to claim 9, wherein the MCK promoter comprises triple muscle specific creatine kinase (tMCK) promoter.

11. The modified adeno-associated virus (AAV) vector for use according to claim 3, wherein the administering is effective to slow disease progression when compared to untreated subjects; or wherein administering is effective to at least temporarily partially reverse paralysis in the subject.

12. A pharmaceutical composition comprising:
a muscle-tropic modified AAV vector packaging an rAAV genome, said rAAV genome engineered to comprise:
(i) a CNTFRα cDNA insert; and
(ii) a promoter; and
a pharmaceutically acceptable excipient.

13. The pharmaceutical composition according to claim 12, comprising a muscle-tropic modified AAV vector packaging an rAAV genome, wherein said rAAV genome is engineered to comprise a CLC cDNA insert and/or a CLF cDNA insert.

14. The pharmaceutical composition according to claim 12 or claim 13 or the modified adeno-associated virus (AAV) vector for use according to any of claims 1 to 11, wherein:
the rAAV genome comprises single-stranded rAAV, self-complementary rAAV, or combinations thereof; and/or
the composition is formulated for intramuscular administration or intravenous administration.

15. The pharmaceutical composition according to claim 12 or claim 13 wherein the promoter is selected from the group consisting of a cytomegalovirus early enhancer element/chicken beta-actin (CAG) promoter and a muscle-specific promoter; optionally wherein the promoter is a muscle specific promoter selected from the group consisting of muscle specific creatine kinase (MCK) promoter, double MCK (dMCK) promoter, and triple MCK (tMCK) promoter.

16. A pharmaceutical composition according to any of claim 12 to 15, for use in a method for treating a patient suffering from a disorder associated with degeneration of motor neuron axons, the method comprising administering to the subject the pharmaceutical composition.

## Patentansprüche

1. Modifizierter Adeno-assoziierter-Virus(adeno-associated virus ― AAV)-Vektor, der ein rekombinanter-AAV(recombinant AAV ― rAAV)-basiertes Genom verpackt, zur Verwendung in einem Verfahren zum Behandeln einer degenerativen Erkrankung eines Motoneurons, wobei das Verfahren Folgendes umfasst: Verabreichen eines modifizierten Adeno-assoziierter-Virus(AAV)-Vektors, der ein rekombinanter-AAV(rAAV)-basiertes Genom verpackt, an ein Subjekt, das an der degenerativen Erkrankung des Motoneurons leidet, wobei das rAAV-basierte Genom ein cDNA-lnsert eines ciliären neurotrophen Faktorrezeptors alpha (ciliary neurotrophic factor receptor alpha ― CNTFRα) umfasst und wobei das Verfahren eine CNTFRa-RNA-Expression in einem Skelettmuskel hochreguliert.

2. Modifizierter Adeno-assoziierter-Virus(AAV)-Vektor zur Verwendung nach Anspruch 1, wobei das Verfahren ferner das Verabreichen eines AAV-Vektors an das Subjekt umfasst, der ein rAAV-basiertes Genom umfasst, das ein CLC-cDNA-Insert umfasst, und/oder wobei das Verfahren ferner das Verabreichen eines AAV-Vektors an das Subjekt umfasst, der ein rAAV-basiertes Genom umfasst, das ein CLF-cDNA-Insert umfasst.

3. Modifizierter Adeno-assoziierter-Virus(AAV)-Vektor zur Verwendung nach Anspruch 1, wobei die Erkrankung Amyotrophe Lateralsklerose (ALS) umfasst.

4. Modifizierter Adeno-assoziierter-Virus(AAV)-Vektor zur Verwendung nach Anspruch 3, wobei ALS eine ALS in einem Spätstadium umfasst; oder wobei die ALS durch Folgendes gekennzeichnet ist: wenigstens eine TDP-43-Mutation; und/oder eine ungewöhnliche zelluläre TDP-43-Verteilung.

5. Modifizierter Adeno-assoziierter-Virus(AAV)-Vektor zur Verwendung nach Anspruch 1, wobei das rAAV-basierte Genom modifiziert ist, um muskeltropisch zu sein.

6. Modifizierter Adeno-assoziierter-Virus(AAV)-Vektor zur Verwendung nach Anspruch 1, wobei das Verfahren Folgendes umfasst:
- Verabreichen des modifizierten Adeno-assoziierter-Virus(AAV)-Vektors an das Subjekt durch nicht-systemische Verabreichung; oder
- Verabreichen des modifizierten Adeno-assoziierter-Virus(AAV)-Vektors an das Subjekt durch nicht-systemische Verabreichung, und wobei die nicht-systemische Verabreichung eine intramuskuläre (IM) Verabreichung an einen oder mehrere Muskeln umfasst, die aus nicht-respiratorischen Skelettmuskeln, respiratorischen Muskeln und Kombinationen davon ausgewählt sind.

7. Modifizierter Adeno-assoziierter-Virus(AAV)-Vektor zur Verwendung nach Anspruch 1, wobei das Verfahren ein Initiieren der Verabreichung des modifizierten Adeno-assoziierter-Virus(AAV)-Vektors an das Subjekt vor, gleichzeitig mit oder nach einem Auftreten von motorischen Symptomen bei dem Subjekt umfasst.

8. Modifizierter Adeno-assoziierter-Virus(AAV)-Vektor zur Verwendung nach Anspruch 1, wobei das rAAV-Genom einen Promotor umfasst, der aus einem Cytomegalovirus-Früh-Enhancer-Element/Hühner-Beta-Actin(chicken beta-actin ― CAG)-Promotor und einem muskelspezifischen Promotor ausgewählt ist.

9. Modifizierter Adeno-assoziierter-Virus(AAV)-Vektor zur Verwendung nach Anspruch 8, wobei der Promotor einen muskelspezifischen Promotor umfasst, der einen muskelspezifische-Kreatinkinase(muscle specific creatine kinase ― MCK)-Promotor umfasst.

10. Modifizierter Adeno-assoziierter-Virus(AAV)-Vektor zur Verwendung nach Anspruch 9, wobei der MCK-Promotor einen dreifache-muskelspezifische-Kreatinkinase(triple muscle specific creatine kinase ― tMCK)-Promotor umfasst.

11. Modifizierter Adeno-assoziierter-Virus(AAV)-Vektor zur Verwendung nach Anspruch 3, wobei das Verabreichen wirksam ist, um ein Fortschreiten der Krankheit im Vergleich zu unbehandelten Subjekten zu verlangsamen; oder wobei das Verabreichen wirksam ist, um eine Lähmung bei dem Subjekt wenigstens vorübergehend teilweise umzukehren.

12. Pharmazeutische Zusammensetzung, die Folgendes umfasst:
einen muskeltropischen modifizierten AAV-Vektor, der ein rAAV-Genom verpackt, wobei das rAAV-Genom manipuliert ist, um Folgendes zu umfassen:
(i) ein CNTFRα-cDNA-lnsert und
(ii) einen Promotor; und
einen pharmazeutisch unbedenklichen Arzneistoffträger.

13. Pharmazeutische Zusammensetzung nach Anspruch 12, die einen muskeltropischen modifizierten AAV-Vektor umfasst, der ein rAAV-Genom verpackt, wobei das rAAV-Genom manipuliert ist, um ein CLC-cDNA-Insert und/oder ein CLF-cDNA-Insert zu umfassen.

14. Pharmazeutische Zusammensetzung nach Anspruch 12 oder 13 oder modifizierter Adeno-assoziierter-Virus(AAV)-Vektor zur Verwendung nach einem der Ansprüche 1 bis 11, wobei:
das rAAV-Genom einen einzelsträngigen rAAV, einen selbstkomplementären rAAV oder Kombinationen davon umfasst; und/oder
die Zusammensetzung für die intramuskuläre oder intravenöse Verabreichung formuliert ist.

15. Pharmazeutische Zusammensetzung nach Anspruch 12 oder 13, wobei der Promotor aus der Gruppe ausgewählt ist, die aus einem Cytomegalovirus-Früh-Enhancer-Element/Hühner-Beta-Actin(CAG)-Promotor und einem muskelspezifischen Promotor besteht; optional wobei der Promotor ein muskelspezifischer Promotor ist, der aus der Gruppe ausgewählt ist, die aus einem muskelspezifische-Kreatinkinase(MCK)-Promotor, einem Doppel-MCK(dMCK)-Promotor und einem dreifache-MCK(tMCK)-Promotor besteht.

16. Pharmazeutische Zusammensetzung nach einem der Ansprüche 12 bis 15 zur Verwendung in einem Verfahren zum Behandeln eines Subjekts, das an einer Erkrankung leidet, die mit einer Degeneration von Motoneuron-Axonen verknüpft ist, wobei das Verfahren das Verabreichen der pharmazeutischen Zusammensetzung an das Subjekt umfasst.

## Revendications

1. Vecteur de virus adéno-associé (VAA) modifié encapsidant un génome à base de VAA recombinant (rVAA), à utiliser dans un procédé de traitement d'un trouble dégénératif des motoneurones, le procédé comprenant : l'administration à un sujet souffrant dudit trouble dégénératif des motoneurones d'un vecteur de virus adéno-associé (VAA) modifié contenant un génome à base de VAA recombinant (rVAA), le génome à base de rVAA comprenant un insert d'ADNc de récepteur du facteur neurotrophique ciliaire (CNTFRα), et le procédé régulant positivement l'expression d'ARN de CNTFRα dans du muscle squelettique.

2. Vecteur de virus adéno-associé (VAA) modifié à utiliser selon la revendication 1, le procédé comprenant en outre l'administration au sujet d'un vecteur de VAA comprenant un génome à base de rVAA comprenant un insert d'ADNc de CLC, et/ou le procédé comprenant en outre l'administration au sujet d'un vecteur de VAA comprenant un génome à base de rVAA comprenant un insert d'ADNc de CLF.

3. Vecteur de virus adéno-associé (VAA) modifié à utiliser selon la revendication 1, le trouble comprenant la sclérose latérale amyotrophique (SLA).

4. Vecteur de virus adéno-associé (VAA) modifié à utiliser selon la revendication 3, la SLA comprenant la SLA de stade avancé ; ou la SLA étant **caractérisée par** l'un ou les deux parmi : au moins une mutation TDP-43 ; et une distribution cellulaire anormale de TDP-43.

5. Vecteur de virus adéno-associé (VAA) modifié à utiliser selon la revendication 1, le génome à base de rVAA étant modifié pour être à tropisme musculaire.

6. Vecteur de virus adéno-associé (VAA) modifié à utiliser selon la revendication 1, le procédé comprenant :
- l'administration du vecteur de virus adéno-associé (VAA) modifié au sujet par administration non-systémique ; ou
- l'administration du vecteur de virus adéno-associé (VAA) modifié au sujet par administration non-systémique et l'administration non-systémique comprenant l'administration intramusculaire (IM) à un ou plusieurs muscles choisis parmi les muscles squelettiques non respiratoires, les muscles respiratoires et leurs combinaisons.

7. Vecteur de virus adéno-associé (VAA) modifié à utiliser selon la revendication 1, le procédé comprenant l'initiation de l'administration du vecteur de virus adéno-associé (VAA) modifié au sujet avant, simultanément ou après l'apparition de symptômes moteurs chez le sujet.

8. Vecteur de virus adéno-associé (VAA) modifié à utiliser selon la revendication 1, le génome de rVAA comprenant un promoteur choisi parmi un élément activateur précoce de cytomégalovirus/bêta-actine de poulet (CAG) et un promoteur spécifique du muscle.

9. Vecteur de virus adéno-associé (VAA) modifié à utiliser selon la revendication 8, le promoteur comprenant un promoteur spécifique du muscle comprenant un promoteur de la créatine kinase spécifique du muscle (MCK).

10. Vecteur de virus adéno-associé (VAA) modifié à utiliser selon la revendication 9, le promoteur MCK comprenant le promoteur de la créatine kinase spécifique du muscle triple (tMCK).

11. Vecteur de virus adéno-associé (VAA) modifié à utiliser selon la revendication 3, l'administration étant efficace pour ralentir la progression de la maladie par rapport aux sujets non traités ; ou l'administration étant efficace pour inverser au moins temporairement la paralysie chez le sujet.

12. Composition pharmaceutique comprenant :
un vecteur de VAA modifié à tropisme musculaire encapsidant un génome de rVAA, ledit génome de rVAA étant conçu pour comprendre :
(i) un insert d'ADNc CNTFRα ; et
(ii) un promoteur ; et
un support pharmaceutiquement acceptable.

13. Composition pharmaceutique selon la revendication 12, comprenant un vecteur de VAA modifié à tropisme musculaire encapsidant un génome de rVAA, dans laquelle ledit génome de rVAA est conçu pour comprendre un insert d'ADNc de CLC et/ou un insert d'ADNc de CLF.

14. Composition pharmaceutique selon la revendication 12 ou la revendication 13 ou le vecteur de virus adéno-associé (VAA) modifié à utiliser selon l'une quelconque des revendications 1 à 11 :
le génome de rVAA comprenant un rVAA simple brin, un rVAA autocomplémentaire ou des combinaisons de ceux-ci ; et/ou
la composition étant formulée pour une administration intramusculaire ou une administration intraveineuse.

15. Composition pharmaceutique selon la revendication 12 ou la revendication 13, le promoteur étant choisi dans le groupe constitué d'un élément activateur précoce du cytomégalovirus/promoteur bêta-actine de poulet (CAG) et d'un promoteur spécifique du muscle ; éventuellement le promoteur étant un promoteur spécifique du muscle choisi dans le groupe constitué du promoteur de la créatine kinase (MCK) spécifique du muscle, du promoteur double MCK (dMCK) et du promoteur triple MCK (tMCK).

16. Composition pharmaceutique selon l'une quelconque des revendications 12 à 15, à utiliser dans un procédé de traitement d'un patient souffrant d'un trouble associé à la dégénérescence des axones des motoneurones, le procédé comprenant l'administration au sujet de la composition pharmaceutique.
